# EUROPÄISCHE PATENTSCHRIFT

(11) **EP 2 692 379 B1**
(45) Veröffentlichungstag und Bekanntmachung des Hinweises auf die Patenterteilung: **16.07.2014**
(21) Anmeldenummer: 12178915.0
(22) Anmeldetag: 01.08.2012
(51) Int. Cl.: A61M 5/315

(54) **Injektionsvorrichtung mit einer Kupplung**
Injection device with a coupling
Dispositif d'injection avec un embrayage

(43) Veröffentlichungstag der Anmeldung: 05.02.2014
(73) Patentinhaber: TecPharma Licensing AG, 3401 Burgdorf (CH)
(72) Erfinder: Hirschel, Jürg, 3007 Bern (CH); Streit, Ursina, 3322 Schönbühl (CH)

(56) Entgegenhaltungen:
- WO-A1-2006/045526
- WO-A1-2009/105909
- WO-A1-2011/101349

## Beschreibung

Die Erfindung betrifft im Allgemeinen eine Sperreinrichtung, insbesondere für eine Antriebs- und/oder Dosiervorrichtung einer oder für eine Injektionsvorrichtung zur Verabreichung eines flüssigen Produkts, insbesondere eines Medikaments, wie zum Beispiel Insulin zur Diabetestherapie. Die Sperreinrichtung dient dazu, ein abtriebsseitig der Sperreinrichtung anliegendes, auf ein erstes Drehglied in eine erste Drehrichtung wirkendes Drehmoment daran zu hindern, ein antriebsseitig der Sperreinrichtung angeordnetes zweites Drehglied und/oder das erste Drehglied in die Richtung zu drehen, in die das abtriebsseitige Drehmoment wirkt. Die Sperreinrichtung erlaubt außerdem, das zweite Drehglied in die Drehrichtung, in die das abtriebsseitige Drehmoment wirkt, zu drehen, wodurch das abtriebsseitige Drehmoment das erste Drehglied in die erste Drehrichtung drehen kann. Die Sperreinrichtung erlaubt außerdem, dass das zweite Drehglied das erste Drehglied in eine zweite Drehrichtung, die dem durch das Drehmomentbeaufschlagungsmittel ausgeübten Drehmoment entgegengesetzt ist, drehen kann.

Aus der WO 2009/105909 A1 ist eine Injektionsvorrichtung bekannt, welche eine als Antriebsfeder dienende Drehfeder aufweist, die durch Drehung eines Dosierknopfs in eine Drehrichtung gespannt und durch Drehung des Dosierknopfs in die entgegengesetzte Drehrichtung entspannt werden kann. Die Injektionsvorrichtung verfügt über eine Rutschkupplung, welche verhindert, dass die vorgespannte Feder den Dosierknopf zurückdreht. Die für diese Funktion erforderlichen Teile sind verhältnismäßig teuer. Bei der gezeigten Vorrichtung handelt es sich um eine wieder verwendbare Vorrichtung, wobei ein entleerter Produktbehälter gegen einen vollen ausgetauscht werden kann. Da für manche Therapien jedoch eine Injektionsvorrichtung von Vorteil ist, die, nachdem der Produktbehälter entleert ist, zusammen mit dem Produktbehälter als Ganzes entsorgt wird (Einweginjektionsvorrichtung), ist es wünschenswert, dass die Injektionsvorrichtung möglichst kostengünstig herstellbar ist.

Es ist eine Aufgabe der Erfindung, eine Sperreinrichtung, insbesondere für eine Injektionsvorrichtung oder eine Antriebs- und Dosiervorrichtung für eine Injektionsvorrichtung, anzugeben, wobei die Sperreinrichtung einerseits die gewünschte Funktion erfüllen soll und andererseits kostengünstig herstellbar ist.

Die Aufgabe wird mit dem Gegenstand des Anspruchs 1 gelöst. Vorteilhafte Weiterentwicklungen ergeben sich aus den abhängigen Ansprüchen, der Beschreibung und den Figuren.

Die Erfindung geht von einer Sperreinrichtung aus, die zwischen einem zweiten, insbesondere eingangsseitigen Drehglied, wie z.B. einem Aufziehglied, und einem ersten, insbesondere ausgangsseitigen Drehglied, wie z.B. einem Kupplungsglied, angeordnet ist. Die Sperreinrichtung bewirkt, dass das erste Drehglied gegen eine Drehung in die erste Drehrichtung, in die ein auf das erste Drehglied wirkendes Drehmoment wirkt, gesperrt ist, wenn das zweite Drehglied unbelastet ist, das heißt frei von äußeren Drehmomenten ist. Durch Aufbringung eines Drehmoments auf das zweite Drehglied wird die Sperrung des ersten Drehglieds in die erste Drehrichtung gelöst, wodurch das erste Drehglied und das zweite Drehglied in die erste Richtung drehbar sind. Durch Aufbringung eines Drehmoments in die der ersten Drehrichtung entgegengesetzte zweite Drehrichtung auf das zweite Drehglied wird das erste Drehglied mit dem zweiten Drehglied mitgedreht.

Die Sperreinrichtung weist ein insbesondere einteiliges Kupplungselement auf, welches zwischen dem ersten Drehglied und dem zweiten Drehglied angeordnet ist. Vorzugsweise sind das erste Drehglied drehfest mit dem Kupplungselement und das zweite Drehglied ebenfalls drehfest mit dem Kupplungselement verbunden, insbesondere daran befestigt oder gebildet. Die Sperre, welche die Drehung des ersten Drehglieds in die erste Drehrichtung verhindert, wenn das zweite Drehglied unbelastet ist, wird dadurch gelöst, dass das Kupplungselement elastisch verformt wird, insbesondere elastisch tordiert wird. Insbesondere kann das zweite Drehglied relativ zu dem ersten Drehglied über das elastische Kupplungselement tordiert, insbesondere um einen Winkel kleiner 30°, insbesondere kleiner 10° tordiert werden.

Die Sperreinrichtung umfasst vorzugsweise eine Hülse, welche über ihren Innenumfang eine Verzahnung aufweist und das tordierbare Kupplungselement umgibt. Das tordierbare Kupplungselement weist ein Eingriffsglied auf, welches in den Innenumfang der Verzahnung eingreift und durch das Tordieren des Kupplungselements aus der Verzahnung ausrastet. Die Hülse kann zum Beispiel ein Gehäuse oder eine zwischen Gehäuse und dem Kupplungselement angeordnete Zwischenhülse sein, die zum Beispiel relativ zu dem Gehäuse drehbar ist.

Das hülsenförmige, insbesondere einstückige Kupplungselement weist einen ersten, insbesondere ringförmigen Abschnitt und einen zweiten, insbesondere ringförmigen Abschnitt auf. Der erste Abschnitt ist mit dem zweiten Abschnitt über mindestens einen elastisch verformbaren Zwischenabschnitt, wie zum Beispiel über zwei Zwischenabschnitte, die zum Beispiel gleichmäßig über den Umfang des Kupplungselements verteilt sind, verbunden. Der Zwischenabschnitt weist das Eingriffsglied auf, welches in die Verzahnung der Hülse eingreift.

Die Sperreinrichtung umfasst ein Drehmomentbeaufschlagungsmittel, welches den ersten Abschnitt des Kupplungselements und/oder das erste Drehglied um die Längsachse mit einem in die erste Drehrichtung gerichteten Drehmoment beaufschlagt, insbesondere permanent beaufschlagt. Das Drehmomentbeaufschlagungsmittel kann zum Beispiel eine Feder, wie zum Beispiel eine Drehfeder sein. Eine Drehfeder kann zum Beispiel eine Wendelfeder oder eine Spiralfeder sein. Das Drehmomentbeaufschlagungsmittel kann zum Beispiel auf das Drehglied wirken, das mit dem ersten Abschnitt verbunden, insbesondere lösbar verbunden oder an dem ersten Abschnitt gebildet ist. Das Eingriffsglied des Kupplungselements wird durch dieses Drehmoment in die Verzahnung der Hülse gedrückt. Der Zwischenabschnitt ist insbesondere so ausgestaltet, dass das Eingriffsglied in die Verzahnung gedrückt wird. Hierdurch wird eine Drehung des Kupplungselements relativ zu der Hülse in die erste Drehrichtung, nämlich in die Drehrichtung, in die das Drehmoment des Drehmomentbeaufschlagungsmittels wirkt, blockiert.

Der zweite Abschnitt des Kupplungselements ist relativ zu dem ersten Abschnitt um die Längsachse in die erste Drehrichtung tordierbar. Dabei wird der mindestens eine Zwischenabschnitt elastisch verformt. Der die Verdrehung in die erste Drehrichtung sichernde Eingriff des Eingriffsglieds in die Verzahnung wird durch Torsion bzw. durch Tordieren des zweiten Abschnitts relativ zum ersten Abschnitt bzw. die Verformung des mindestens einen Zwischenabschnitts gelöst. Somit ist eine Drehung des Kupplungselements relativ zu der Hülse in die erste Drehrichtung, insbesondere zumindest um ein Winkelinkrement, freigegeben.

Die Innenverzahnung der Hülse weist eine Vielzahl über den Innenumfang, insbesondere gleichmäßig verteilte Zähne auf. Der Winkelabstand zwischen zwei benachbarten Zähnen kann als Winkelinkrement bezeichnet werden. Das Kupplungselement ist relativ zu der Hülse in die erste Drehrichtung und zumindest oder vorzugsweise um ein Winkelinkrement drehbar. Das Eingriffsglied kann durch Tordieren des zweiten Abschnitts in die erste Drehrichtung aus einem Zahn der Verzahnung ausrasten oder zumindest so gelockert werden, dass das Drehmomentbeaufschlagungsmittel das Eingriffsglied über den Zahn der Verzahnung bewegt, und kann in einen benachbarten, insbesondere nächst benachbarten Zahn einrasten, wodurch die Drehung des Kupplungselements relativ zu der Hülse in die erste Drehrichtung wieder gesperrt wird. Wird der zweite Abschnitt relativ zu dem ersten Abschnitt wieder in die erste Drehrichtung tordiert, wiederholt sich der Vorgang. Insbesondere rastet das Eingriffsglied aus diesem Zahn aus und in den nächst benachbarten Zahn ein, wobei das Kupplungselement relativ zu der Hülse wieder um ein Winkelinkrement in die erste Drehrichtung gedreht wird. Ist die Sperreinrichtung Teil einer Dosiervorrichtung für eine Injektionsvorrichtung kann ein Winkelinkrement einem gewünschten Dosierschritt, wie zum Beispiel der Dosis 1 IU oder 2 IU entsprechen.

Vorzugsweise wird der zweite Abschnitt relativ zu dem ersten Abschnitt per Hand, das heißt per Muskelkraft durch einen Verwender relativ zu dem ersten Abschnitt in die erste Drehrichtung tordiert, wobei sich der Zwischenabschnitt elastisch verformt und das Eingriffsglied aus dem Zahn ausrastet oder der Eingriff entsprechend gelockert wird. Das in die erste Drehrichtung wirkende Drehmoment des Drehmomentbeaufschlagungsmittels oder/und die Elastizität des Zwischenabschnitts kann den ersten Abschnitt relativ zu dem zweiten Abschnitt in die erste Drehrichtung tordieren, wodurch der Zwischenabschnitt zurückverformt wird und das Eingriffsglied in den nächsten Zahn der Verzahnung sperrend eingreift.

Ist das Drehmomentbeaufschlagungsmittel eine Feder, insbesondere eine Drehfeder, kann diese durch Drehung des Kupplungselements in die erste Drehrichtung entspannt und durch Drehung des Kupplungselements in die zweite Drehrichtung gespannt werden.

In bevorzugten Ausführungen kann das Kupplungselement relativ zu der Hülse in die zweite, der ersten Drehrichtung entgegengesetzte Drehrichtung drehbar sein oder gedreht werden, wobei das Eingriffsglied des Kupplungselements über die Verzahnung der Hülse rastet.

Alternativ kann die Hülse, die auch als erste Hülse bezeichnet werden kann und in deren Verzahnung das Eingriffsglied des Zwischenabschnitts eingreift, ein elastisch angeordnetes Eingriffsglied aufweisen, welches in eine Verzahnung, insbesondere Innenverzahnung, einer die Hülse umgebenden zweiten Hülse, wie zu Beispiel einem hülsenförmigen Gehäuse, eingreift. Das Eingriffsglied der zweiten Hülse kann über die Verzahnung der ersten Hülse rasten, wenn die erste Hülse relativ zu der zweiten Hülse in die zweite Drehrichtung gedreht wird. Eine Drehung der ersten Hülse relativ zu der sie umgebenden zweiten Hülse in die erste Drehrichtung ist vorzugsweise gesperrt. Zwischen der ersten Hülse und der zweiten Hülse ist somit eine unidirektionale Kupplung gebildet, welche die Drehung nur in eine Drehrichtung, nämlich die zweite Drehrichtung zulässt. Das Kupplungselement kann relativ zu der ersten Hülse in die zweite Drehrichtung drehfest sein. Wird das Kupplungselement in die zweite Drehrichtung gedreht, wird die erste Hülse mit dem Kupplungselement mitgedreht, wobei die erste Hülse relativ zu der zweiten Hülse in die zweite Drehrichtung gedreht wird.

In bevorzugten Ausführungsformen weist die Verzahnung eine Vielzahl Zähne auf, wobei ein oder mehrere, insbesondere jeder dieser Zähne eine in eine Umfangsrichtung weisende erste Flanke und eine entgegengesetzt zu der ersten Flanke in Umfangsrichtung weisende zweite Flanke aufweisen. Die erste Flanke ist vorzugsweise steiler ausgebildet als die zweite Flanke. Hierdurch wird eine sägezahnförmige Form des jeweiligen Zahns der Verzahnung gebildet. Vorzugsweise drückt das Eingriffsglied gegen die erste, steilere Flanke, wenn das Kupplungselement mit dem Drehmoment des Drehmomentbeaufschlagungsmittels beaufschlagt ist. Die zweite, flacher ausgebildete Flanke ermöglicht dem Kupplungselement relativ zu der Hülse in die zweite Drehrichtung gedreht zu werden, wobei das Eingriffsglied an der flachen Flanke abgleitet und aus dem Eingriff mit dem jeweiligen Zahn rastet.

Vorzugsweise ist das Eingriffsglied zahnförmig, insbesondere sägezahnförmig. Das Eingriffsglied kann eine in die erste Drehrichtung weisende erste Flanke und eine in die zweite Drehrichtung weisende zweite Flanke aufweisen, wobei die erste Flanke steiler als die zweite Flanke ausgebildet ist. Durch die steile erste Flanke wird eine Drehung des Kupplungselements relativ zu der Hülse verhindert, während die flache zweite Flanke eine Drehung des Kupplungselements relativ zu der Hülse in die zweite Drehrichtung erlaubt.

Vorzugsweise mündet der Zwischenabschnitt mit einem seiner Enden in den ersten Abschnitt und mit dem anderen seiner beiden Enden in den zweiten Abschnitt. Zum Beispiel können die Enden des Zwischenabschnitts abgewinkelt sein. Das Eingriffsglied ist zwischen den beiden Enden vorzugsweise mittig angeordnet.

Der Zwischenabschnitt kann länglich und/oder stegförmig ausgestaltet sein, wobei er sich seiner Länge nach über einen Teil des Umfangs des Kupplungsabschnitts erstreckt.

In besonders bevorzugten Ausführungen kann sich der Zwischenabschnitt von dem ersten Abschnitt in die erste Drehrichtung erstrecken und/oder von dem zweiten Abschnitt entgegen der ersten Drehrichtung, das heißt in die zweite Drehrichtung erstrecken. Dadurch, dass sich der Zwischenabschnitt von dem ersten Abschnitt in die erste Drehrichtung erstreckt, wird vorteilhaft eine Selbstverstärkung des Sperreingriffs des Eingriffsglieds in die Verzahnung erreicht. Das selbst verstärkende Prinzip ist im sehr weiten Sinne mit einer auflaufenden Bremsbacke einer Trommelbremse vergleichbar.

Um die elastischen Eigenschaften des Zwischenabschnitts einstellen zu können, kann der Zwischenabschnitt eine geringere Wandstärke aufweisen als der erste und/oder zweite Abschnitt des Kupplungselements.

Insbesondere können der erste Abschnitt und/oder der zweite Abschnitt jeweils eine Verdrehsicherungsstruktur aufweisen, wodurch ein erstes oder/und zweites Drehglied mit dem jeweiligen Abschnitt drehfest verbindbar ist. Die Verdrehsicherungsstruktur kann zum Beispiel eine Verzahnung oder eine Nut am Innenumfang des ersten oder zweiten Abschnitts sein. Zum Beispiel kann die Verdrehsicherungsstruktur eine Kupplungsstruktur bilden, die Teil einer lösbaren Kupplung ist. Zum Beispiel kann ein Drehglied, wie z.B. das erste Drehglied, eine Kupplungsstruktur aufweisen, welche in die Verdrehsicherungsstruktur des z.B. ersten Abschnitts des Kupplungselements eingreift, wodurch die Kupplung geschlossen ist. Das Drehglied kann entlang der Längs- oder Drehachse relativ zu dem Kupplungselement verschiebbar sein, so dass die Kupplungsstruktur des Drehglieds aus dem Eingriff mit der Verdrehsicherungsstruktur, beispielsweise des ersten Abschnitts gerät, wodurch das Drehmomentbeaufschlagungsmittel das erste Drehglied relativ zu dem Kupplungselement zum Beispiel in die erste Drehrichtung verdrehen kann.

In einer vorteilhaften Weiterbildung kann das zweite Drehglied so mit dem Kupplungselement verbunden sein, dass das zweite Drehglied gegen einen ersten Drehanschlag des Kupplungselements gedrückt wird, wenn es in die erste Drehrichtung gedreht wird, und gegen einen in die entgegengesetzte Drehrichtung wirkenden zweiten Drehanschlag gedrückt wird, wenn es in die zweite Drehrichtung gedreht wird, wobei der erste Drehanschlag an dem zweiten Abschnitt und der zweite Drehanschlag an dem ersten Abschnitt oder dem Zwischenabschnitt, insbesondere zwischen dem Eingriffsglied und dem ersten Abschnitt, oder in dem Bereich, wo der Zwischenabschnitt in den ersten Abschnitt mündet, gebildet ist. Hierdurch wird vorteilhaft bewirkt, dass das auf das zweite Drehglied wirkende Drehmoment an den ersten Abschnitt übertragen wird, ohne über den gesamten Zwischenabschnitt laufen zu müssen, wenn das zweite Drehglied in die zweite Drehrichtung gedreht wird. Durch Umgehung des Zwischenabschnitts hinsichtlich der Drehmomenteinleitung in das Kupplungselement, kann das Kupplungselement noch einfacher in die zweite Drehrichtung verdreht werden. Wird der zweite Abschnitt relativ zu dem ersten Abschnitt tordiert, wird das zweite Drehglied, insbesondere ein mit dem zweiten Drehanschlag zusammenwirkender Gegenanschlag, von dem zweiten Drehanschlag wegbewegt.

Zum Beispiel kann das zweite Drehglied permanent oder in beide Drehrichtungen drehfest mit dem zweiten Abschnitt des Kupplungselements verbunden sein, insbesondere mittels einer geeigneten Welle-Nabe-Verbindung, wie z.B. einer Nut, die am Innenumfang des zweiten Abschnitts gebildet ist, in welche eine Abragung des zweiten Drehglieds eingreift. Eine der Nutflanken der Nut kann den ersten Drehanschlag bilden, wobei die Abragung des zweiten Dosierglieds den entsprechenden Gegenanschlag bilden kann. Die in die Nut eingreifende Abragung oder eine weitere Abragung kann z.B. den Gegenanschlag für den zweiten Drehanschlag bilden.

Der erste Drehanschlag kann allgemein am Innenumfang des zweiten Abschnitts gebildet sein, wobei der entsprechende Gegenanschlag bei Drehung des zweiten Drehglieds in die erste Drehrichtung zu dem ersten Drehanschlag hin bewegt und schließlich dagegen gedrückt wird. Optional liegt der Gegenanschlag an dem ersten Drehanschlag an, wenn das zweite Drehglied drehmomentunbeaufschlagt ist. Der für den zweiten Drehanschlag entsprechende Gegenanschlag kann bei Drehung des zweiten Drehglieds in die zweite Drehrichtung zu dem zweiten Drehanschlag hin bewegt und schließlich dagegen gedrückt werden. Optional kann der Gegenanschlag an dem zweiten Anschlag anliegen, wenn das zweite Drehglied drehmomentunbeaufschlagt ist. Alternativ zu einer in beide Drehrichtungen drehfesten Verbindung kann das zweite Drehglied, insbesondere in einem beschränkten Maß, wie z.B. wenige Winkelgrade, relativ zu dem zweiten Abschnitt verdrehbar sein.

Bevorzugt ist die Sperreinrichtung Teil einer Antriebs- und/oder Dosiervorrichtung für eine Injektionsvorrichtung oder einer Injektionsvorrichtung. Die Injektionsvorrichtung dient zur Verabreichung eines flüssigen Produkts, insbesondere eines Medikaments.

Die Antriebs- und Dosiervorrichtung umfasst vorzugsweise ein vom Verwender der Vorrichtung greifbares Aufziehglied (zweites Drehglied) und eine mittels Drehung des Aufziehglieds spannbare Ausschüttfeder, die als Drehmomentbeaufschlagungsmittel dient, und die vorzugsweise bereits leicht vorgespannt ist. Die Ausschüttfeder speichert vorzugsweise die für die Produktausschüttung erforderliche Energie. Die erfindungsgemäße Sperreinrichtung kann zwischen, insbesondere kinematisch und/oder geometrisch zwischen dem Aufziehglied und der Ausschüttfeder angeordnet sein. Durch die zwischen dem Aufziehglied und der Ausschüttfeder angeordnete Sperreinrichtung wird bewirkt, dass eine Drehung des Aufziehglieds in die erste Drehrichtung die Ausschüttfeder entspannt und eine Drehung des Aufziehglieds in die zweite Drehrichtung die Ausschüttfeder spannt. Ferner wird verhindert, dass die gespannte Ausschüttfeder sich entspannt oder das Aufziehglied zurückdreht, wenn auf das Aufziehglied kein äußeres Drehmoment aufgebracht wird bzw. das Aufziehglied unbeaufschlagt ist.

In bevorzugten Ausführungsformen kann mit der Antriebs- und/oder Dosiervorrichtung mittels Drehung des Aufziehglieds, insbesondere in die erste und/oder zweite Drehrichtung, eine zu verabreichende Produktdosis einstellbar sein. Durch Drehung des Aufziehglieds in die erste Drehrichtung kann die zu verabreichende Produktdosis verringert werden, wobei durch Drehung des Aufziehglieds in die zweite Drehrichtung die Produktdosis erhöht werden kann. Insbesondere umfasst die Vorrichtung ein Dosisanzeigeelement, welches so mit dem Aufziehglied gekoppelt ist, dass eine Drehung des Aufziehglieds eine Drehung des Dosisanzeigeelements insbesondere relativ zu einem Gehäuse oder einer Zeigeeinrichtung, bewirkt. Das Dosisanzeigeelement kann zum Beispiel hülsenförmig, insbesondere als Dosisanzeigetrommel ausgestaltet sein. Das Dosisanzeigeelement kann an seinem Umfang eine zum Beispiel wendelförmige Skala aufweisen, die durch wendelförmig aneinander gereihte Dosiswerte gebildet wird. Mit der Zeigeeinrichtung, die zum Beispiel an dem Gehäuse gebildet ist, kann an der Skala der eingestellte oder zu verabreichende Dosiswert abgelesen werden.

Vorzugsweise wird mit der Drehung des Dosisanzeigeelements die Ausschüttfeder gespannt oder entspannt.

Die Ausschüttfeder kann mit einem Vortriebsglied, insbesondere einer Kolbenstange gekoppelt sein oder werden, wobei die Ausschüttfeder die für den Vortrieb des Vortriebsglieds in Ausschüttrichtung erforderliche Energie speichert und, insbesondere durch Betätigung eines Betätigungsglieds, abgibt. Das Vortriebsglied kann auf einen Kolben eines Produktbehälters, wie zum Beispiel einer Karpule drücken und den Kolben des Produktbehälters verschieben.

Sofern das Aufziehglied auch zur Einstellung der Produktdosis dient, kann es auch als Dosierglied oder als Dosier- und Aufziehglied bezeichnet werden.

Vorzugsweise kann die Antriebs- und/oder Dosiervorrichtung eine erste Kupplung aufweisen, die insbesondere kinematisch zwischen dem ersten Drehglied oder dem Kupplungselement, und der Ausschüttfeder angeordnet ist. Die geschlossene Kupplung kann das zwischen der Ausschüttfeder und dem Kupplungselement angeordnete erste Drehglied, insbesondere Kupplungsglied, drehfest mit dem Kupplungselement verbinden, wobei das erste Drehglied bei geschlossener Kupplung relativ zu dem Vortriebsglied drehbar oder nicht drehbar ist. Die Antriebs- und Dosiervorrichtung kann ferner ein für die Produktausschüttung betätigbares Betätigungsglied, insbesondere einen Betätigungsknopf, der zum Beispiel am hinteren Ende der Antriebs- und Dosiervorrichtung angeordnet ist, aufweisen, wobei die Kupplung bei unbetätigtem Betätigungsglied geschlossen und bei betätigtem Betätigungsglied geöffnet ist oder durch Betätigung des Betätigungsglieds geöfmet wird. Das Betätigungsglied kann gegen die Kraft einer Kupplungs- oder Rücksetzfeder betätigt werden. Die Kupplungs- und/oder Rücksetzfeder bewirkt, dass die Kupplung geschlossen ist, wenn das Betätigungsglied unbetätigt ist

Insbesondere kann das Vortriebsglied so, wie z.B. mit einer zweiten Kupplung, mit dem zwischen dem Kupplungselement und der Antriebsfeder angeordneten Kupplungsglied oder Drehglied verbunden oder verbindbar sein, dass eine Drehung des Kupplungs- oder Drehglieds in die erste Drehrichtung eine Bewegung des Vortriebsglieds entlang der Längsachse in distale Richtung bewirkt.

Insbesondere kann die Antriebs- und/oder Dosiervorrichtung eine zweite Kupplung aufweisen, welche bei unbetätigtem Betätigungsglied geöffnet und bei betätigtem Betätigungsglied geschlossen ist oder durch Betätigung des Betätigungsglieds geschlossen wird. Die geschlossene zweite Kupplung verbindet das Kupplungs- oder Drehglied und das Vortriebsglied so miteinander dass eine Drehung des Kupplungs- oder Drehglieds die Bewegung des Vortriebsglieds in distale Richtung bewirkt.

Das Kupplungselement ist vorzugsweise ein aus Kunststoff, wie z.B. POM oder PEEK mit oder ohne Zuschlagstoffe wie beispielsweise Glas-, Mineral-oder Karbonfasern hergestelltes Teil, insbesondere Spritzgussteil. Weiter kann das Kupplungselement mit vorteilhafter Elastizität bei gleichzeitig hoher Kriechfestigkeit in Zweikomponententechnik gespritzt oder/und durch Einlegen und Umspritzen eines Elements, z.B. Einlegeteils aus Stahl, z.B. Federstahl oder Buntmetall, z.B. Bronze gefertigt sein.

Die Erfindung wurde anhand mehrerer bevorzugter Ausführungen beschrieben. Im Folgenden werden bevorzugte Ausführungen anhand von Figuren beschrieben. Die dabei offenbarten Merkmale bilden den erfindungsgemäßen Gegenstand einzeln und in jeglicher Merkmalskombination, insbesondere auch mit den oben beschriebenen Merkmalen vorteilhaft weiter. Es zeigen:
- Figur 1: eine Explosionsdarstellung der Einzelteile einer Injektionsvorrichtung mit einer erfindungsgemäßen Sperreinrichtung nach einer ersten Ausführungsform,
- Figur 2: die Darstellung aus Figur 1, wobei die Einzelteile im Schnitt dargestellt sind,
- Figuren 3a bis 3d: verschiedene Ansichten einer aus den Einzelteilen der Figuren 1 und 2 zusammengesetzten Injektionsvorrichtung in einem Ausgangs- oder Auslieferungszustand,
- Figuren 4a bis 4d: verschiedene Ansichten der Vorrichtung aus den Figuren 3a bis 3d mit einer maximal eingestellten Dosis,
- Figuren 5 bis 5d: verschiedene Ansichten der Vorrichtung aus den Figuren 3a bis 3d nach Ausschüttung der eingestellten Produktdosis,
- Figuren 6a bis 6d: verschiedene Ansichten der Vorrichtung aus den Figuren 3a bis 3d in einem Zustand, bei dem die in dem Produktbehälter enthaltene Produktdosis geringer als die mit der Vorrichtung maximal einstellbare Dosis ist,
- Figur 7: ein Kupplungselement der Sperreinrichtung der Injektionsvorrichtung nach den Figuren 1 bis 6d und im weiteren Sinne auch für die Sperreinrichtung nach Figur 8 und
- Figur 8: verschiedene Ansichten einer zweiten Ausführungsform einer Sperreinrichtung.

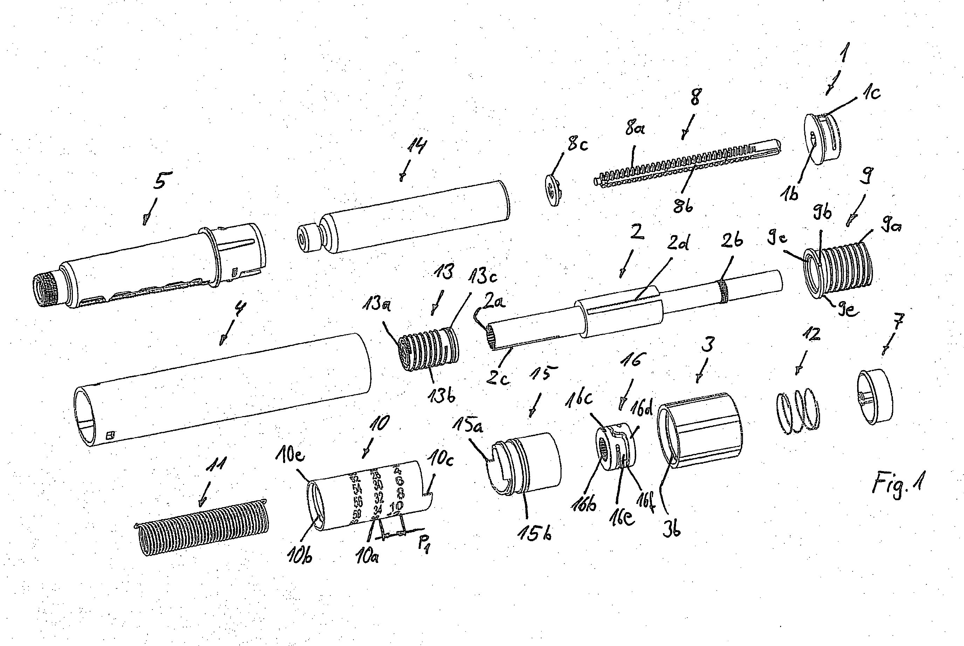

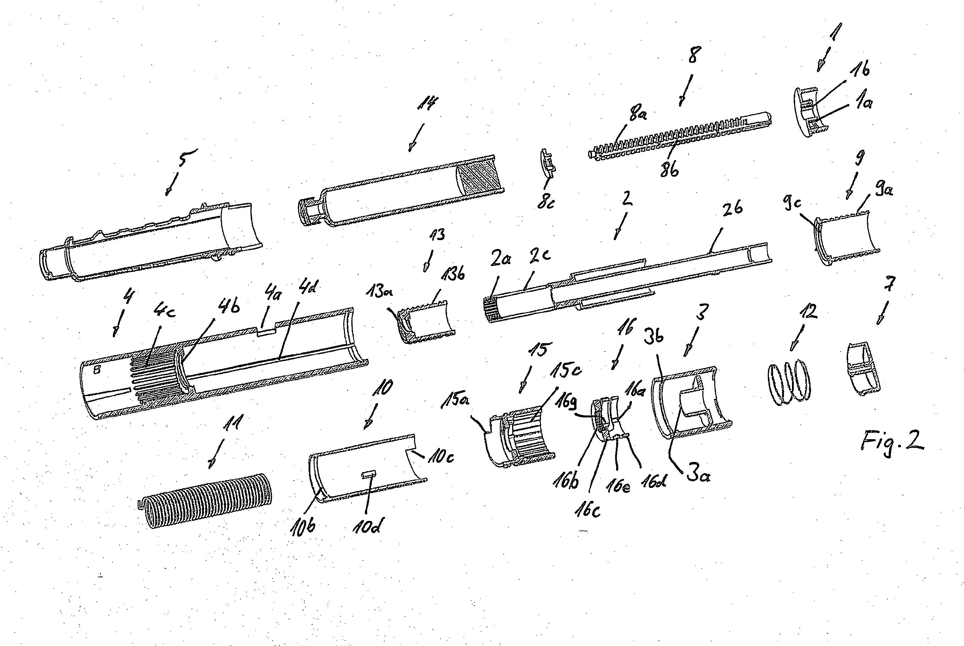

Die in den Figuren 1 bis 6d gezeigte Injektionsvorrichtung weist ein hülsenförmiges Gehäuse 4 auf, in dem ein fensterartiger Durchbruch zur Bildung einer Zeigeeinrichtung 4a angeordnet ist. An dem distalen, d.h. vorderen Ende des Gehäuses 4 ist ein Produktbehälterhalter 5 formschlüssig, vorzugsweise unlösbar befestigt, insbesondere verschnappt, welcher einen Produktbehälter 14 in der Gestalt einer Karpule aufnimmt. Die Karpule weist ein zylindrisches Gehäuse auf, in dem ein Kolben verschiebbar angeordnet ist. Am distalen Ende weist die Karpule ein mit einer Nadel durchstechbares Septum auf. Zwischen dem Septum und dem Kolben befindet sich das zu verabreichende Produkt. Durch Verschiebung des Kolbens Richtung Septum wird das Produkt aus dem Produktbehälter 14 verdrängt. An dem proximalen Ende des Produktbehälterhalters 5 ist ein Gewinde oder ein Bajonettverschluss gebildet, an dem die Nadel befestigbar ist. Auf den Produktbehälterhalter 5 kann eine Kappe 6 (nicht gezeigt) abnehmbar aufgesteckt werden.

An dem proximalen, d.h. hinteren Ende des Gehäuses 4 ist ein relativ zu dem Gehäuse 4 drehbares Aufziehglied 3, welches als Dosierglied 3 bezeichnet wird, angeordnet. Das Dosierglied 3 bildet eine äußere Oberfläche der Vorrichtung und ist vom Verwender der Vorrichtung ergreifbar und relativ zu dem Gehäuse 4 verdrehbar. Eine Drehung des Dosierglieds 3 in eine erste Drehrichtung bewirkt eine Verringerung der Dosis, wobei die Drehung des Dosierglieds 3 in eine zweite Drehrichtung eine Erhöhung der Dosis bewirkt. Das Dosierglied 3 ist axialfest mit dem Gehäuse 4 verbunden. An dem Gehäuse 4 ist ein hülsenförmiger Gehäuseeinsatz 15 dreh- und axialfest befestigt, der somit gehäusefest ist und dem Gehäuse 4 zugerechnet werden kann. Der Gehäuseeinsatz 15 weist eine Ringnut 15b auf, in die ein Ringsteg 3b am inneren Umfang des Dosierglieds 3 einrastet, wodurch das Dosierglied 3 drehbar und axialfest mit dem Gehäuseeinsatz 15 verbunden ist.

Insbesondere am distalen Ende des Gehäuseeinsatzes 15 ist ein in Umfangsrichtung wirkender Maximaldosisgegenanschlag 15a für einen Maximaldosisanschlag 10c eines Dosisanzeigeelements 10 gebildet.

Über den inneren Umfang weist der hülsenförmige Gehäuseeinsatz 15 eine Verzahnung 15c auf. Der Gehäuseeinsatz 15 umgibt ein einstückiges Kupplungselement 16, das vorzugsweise aus Kunststoff hergestellt und/oder ein Spritzgussteil ist. Das Kupplungselement 16 weist einen ersten Abschnitt 16c und einen zweiten Abschnitt 16d, der mit dem zweiten Abschnitt 16d über einen elastisch verformbaren Zwischenabschnitt 16e verbunden ist, auf. Der Zwischenabschnitt 16e mündet mit einem Ende in den ersten Abschnitt 16c und mit dem anderen Ende in den zweiten Abschnitt 16d. Der Zwischenabschnitt 16e erstreckt sich von dem ersten Abschnitt 16c in die erste Drehrichtung und von dem zweiten Abschnitt 16d entgegen der ersten Drehrichtung, das heißt in die zweite Drehrichtung. Der Zwischenabschnitt 16e ist vorzugsweise länglich und erstreckt sich zumindest über einen Teil des Umfangs des Kupplungselements 16. Das Kupplungselement 16 weist zwei solche Zwischenabschnitte 16e auf, die insbesondere um 180° versetzt über den Umfang angeordnet sind.

Der oder die Zwischenabschnitte 16e weisen insbesondere jeweils ein Eingriffsglied 16f auf, welches in die Verzahnung 15c der Hülse 15 eingreift. Das Eingriffsglied 16f ist insbesondere als ein radial vom Umfang des Kupplungselements 16 nach außen ragender Zahn oder Nocken gebildet.

Das Eingriffsglied 16f weist eine in die erste Drehrichtung weisende, erste Zahnflanke und eine in die zweite Drehrichtung weisende, zweite Zahnflanke auf. Die erste und zweite Zahnflanke sind unterschiedlich steil angeordnet, so dass das Eingriffsglied 16f sägezahnförmig ist. Die erste Flanke ist vorzugsweise steiler als die zweite Flanke angeordnet.

Das Eingriffsglied 16f ist vorzugsweise zwischen den Enden des Zwischenabschnitts 16e, vorzugsweise mittig, angeordnet.

Die Verzahnung 15c weist eine Vielzahl über den im Umfang verteilte Zähne auf. Ein Zahn oder mehrere dieser Zähne, insbesondere jeder dieser Zähne können zum Beispiel sägezahnförmig gebildet sein. Sie können eine in Umfangsrichtung weisende erste Flanke und eine entgegengesetzt zu der ersten Flanke in Umfangsrichtung weisende zweite Flanke aufweisen, wobei die erste Flanke steiler als die zweite Flanke ausgebildet ist. Vorzugsweise bildet die erste Flanke eine Gegenflanke für die erste Flanke des Eingriffsglieds 16f.

Eine als Drehmomentbeaufschlagungsmittel dienende, in diesem Beispiel als Wendelfeder ausgestaltete Drehfeder 11 stützt sich mit einem, insbesondere dem distalen Ende an einem relativ zu dem Gehäuse 4 drehbaren Drehglied 2, das in diesem Beispiel als Kupplungsglied 2 dient und daher so bezeichnet wird, ab und mit dem anderen, insbesondere dem proximalen Ende gehäusefest, insbesondere an dem Gehäuseeinsatz 15, der dem Gehäuse 4 zugeordnet werden kann, ab. Eine Drehung des Kupplungsglieds 2 in die zweite Drehrichtung bewirkt ein Spannen der Drehfeder 11, wobei eine Drehung in die erste Drehrichtung ein Entspannen der Feder 11 bewirkt. Das Kupplungsglied 2 ist vorzugsweise über eine lösbare Kupplung 2b, 16b, insbesondere erste Kupplung, im Wesentlichen verdrehgesichert mit dem Dosierglied 3 verbunden. Das Kupplungsglied 2 weist zur Bildung der ersten Kupplung eine Kupplungsstruktur 2b auf. Der erste Abschnitt 16c des Kupplungselements 16 weist zur Bildung der ersten Kupplung 2b, 16b eine Kupplungsstruktur 16b auf, welche bei geschlossener Kupplung 2b, 16b in einem verdrehgesicherten Eingriff mit der Kupplungsstruktur 2b ist.

Das Dosierglied 3 kann in einer ersten Variante permanent oder in beide Drehrichtungen drehfest mit dem zweiten Abschnitt 16d des Kupplungselements 16 verbunden sein. Für die verdrehgesicherte Verbindung weist der zweite Abschnitt 16d an seinem Innenumfang eine Nut 16a auf, in welche eine Abragung 3a des Dosierglieds 3 eingreift. Eine Nutflanke der Nut 16a bildet einen ersten Drehanschlag 16h, gegen den die Abragung 3a gegenanschlagsartig gedrückt wird, wenn das Dosierglied 3 in die erste Drehrichtung gedreht wird. Optional kann an dem ersten Abschnitt 16c oder dem Zwischenabschnitt 16e, insbesondere zwischen Eingriffsglied 16f und erstem Abschnitt 16c oder dort, wo der Zwischenabschnitt 16e in den ersten Abschnitt 16c mündet, ein als Drehanschlag wirkender Anschlag 16g, der auch als zweiter Drehanschlag bezeichnet werden kann, gebildet sein, gegen den ein Gegenanschlag des Dosierglieds 3, der z.B. von der rippenförmigen Abragung 3a oder einer anderen Abragung gebildet sein kann, gedrückt wird, wenn das Dosierglied 3 in die zweite Drehrichtung gedreht wird. Hierdurch wird das auf das Dosierglied 3 wirkende Drehmoment an den ersten Abschnitt 16c übertragen, ohne über den gesamten Zwischenabschnitt 16e laufen zu müssen. Hierdurch kann das Kupplungselement 16 noch einfacher relativ zu der Verzahnung 15c in die zweite Drehrichtung verdreht werden.

In einer zweiten Variante kann das Dosierglied 3 in die erste Drehrichtung drehfest mit dem zweiten Abschnitt 16d des Kupplungselements 16 und in die zweite Drehrichtung drehfest mit dem ersten Abschnitt 16c oder dem Verbindungsabschnitt 16e verbunden sein. Z.B. kann das Dosierglied 3 somit, insbesondere in einem beschränkten Maß, wie z.B. wenige Winkelgrade, relativ zu dem zweiten Abschnitt 16d verdrehbar sein. Für diese Verbindung weist der zweite Abschnitt 16d an seinem Innenumfang den ersten Anschlag 16h auf, an welchen ein Gegenanschlag, insbesondere eine Abragung oder der Längssteg 3a des Dosierglieds 3, in die erste Drehrichtung anschlägt. Weiter weist der Zwischenabschnitt 16e im Bereich der Verbindungsstelle zum ersten Abschnitt 16c den zweiten Anschlag 16g auf, an welchen ein Gegenanschlag, insbesondere die Abragung oder der Längssteg 3a, in die zweite Drehrichtung anschlägt.

Durch die zwei Varianten wird bewirkt, dass das durch das Dosierglied 3 auf das Kupplungselement 16 ausgeübte Drehmoment über den gesamten Verbindungsabschnitt 16e läuft, wenn das Dosierglied 3 in die erste Drehrichtung gedreht wird, so dass sich der Verbindungsabschnitt 16e elastisch verformen kann. Ferner wird bewirkt, dass das durch das Dosierglied 3 auf das Kupplungselement 16 ausgeübte Drehmoment an dem oder in den ersten Abschnitt 16d, ohne über den gesamten Verbindungsabschnitt 16e laufen zu müssen, eingeleitet wird, wenn das Dosierglied 3 in die erste Drehrichtung gedreht wird.

Die Drehfeder 11 beaufschlagt den ersten Abschnitt 16c des Kupplungselements 16 um die Längsachse L mit einem in die erste Drehrichtung gerichteten Drehmoment. Die Höhe des Drehmoments hängt davon ab, wie stark die Drehfeder 11 vorgespannt ist. Das Eingriffsglied 16f wird durch das in die erste Drehrichtung wirkende Drehmoment in die Verzahnung 15c gedrückt, so dass eine Drehung des Kupplungselements 16 relativ zu dem Gehäuseeinsatz 15 in die erste Drehrichtung blockiert wird. Damit das Eingriffsglied 16f sicher in die Verzahnung 15c gedrückt wird, erstreckt sich der Zwischenabschnitt 16e vorteilhaft von dem ersten Abschnitt 16c in Umfangsrichtung in die ersten Drehrichtung. Als alternative oder zusätzliche Maßnahme können die erste Flanke des Eingriffsglieds 16f und die erste Flanke eines der Zähne der Verzahnung 15c so aufeinander abgestimmt werden, dass zwischen dem Eingriffsglied 16f und der Verzahnung 15c eine Selbsthemmung auftrifft, das heißt, dass auch bei einem sehr hohen Drehmoment das Eingriffsglied 16f nicht aus der Verzahnung 15c gedrückt wird. Die Flanken können auch so aufeinander abgestimmt sein, dass das Eingriffsglied 16f in den Eingriff mit der Verzahnung 15c hineingezogen wird.

Der zweite Abschnitt 16d des Kupplungselements 16 ist relativ zu dem ersten Abschnitt 16c um die Längsachse L in die erste Drehrichtung, insbesondere elastisch, tordierbar, insbesondere mittels Drehung des Dosierglieds 3 in die erste Drehrichtung. Die Tordierbarkeit wird durch den elastischen Zwischenabschnitt 16e bereitgestellt. Durch die Torsion des zweiten Abschnitts 16d relativ zu dem ersten Abschnitt 16c wird der die Verdrehung in die erste Drehrichtung sichernde Eingriff des Eingriffsglieds 16f in die Verzahnung 15c gelöst. Durch die Torsion wird das Eingriffsglied 16f ein kleines Stück nach innen, das heißt zur Drehachse hin ausgelenkt, so dass das Eingriffsglied 16f aus dem verdrehgesicherten Eingriff gelöst wird. Somit ist eine Drehung des Kupplungselements 16 relativ zu dem Gehäuseeinsatz 15 in die erste Drehrichtung freigegeben. Das Eingriffsglied 16f überspringt somit den Zahn der Verzahnung 15c, mit dem es im verdrehgesicherten Eingriff war. Hierbei wird es von dem in die erste Drehrichtung wirkenden Drehmoment der Drehfeder 11 angetrieben. Das Eingriffsglied 16f rastet in den nächsten, insbesondere nächst benachbarten Zahn ein. Somit lässt sich das Kupplungselement 16 relativ zu dem Gehäuseeinsatz 15 inkrementweise oder um ein Winkelinkrement entsprechend der Zahnteilung der Verzahnung 15c in die erste Drehrichtung drehen.

Hierdurch lässt sich vorteilhaft mit einem einfach und kostengünstig gestalteten Bauteil, nämlich dem hülsenförmigen Kupplungselement 16 die Drehfeder 11 spannen und entspannen und das Entspannen der Drehfeder 11 verhindern.

Durch Drehen des Dosierglieds 3 in die zweite Drehrichtung rastet das Eingriffsglied 16f über die Zähne der Verzahnung 15c. Durch die geschlossene erste Kupplung 2b, 16b wird die Feder 11 gespannt.

Um die gewünschte Elastizität des Zwischenabschnitts 16e zu erhalten, kann der Zwischenabschnitt 16e eine andere Wandstärke als der erste und/oder zweite Abschnitt 16b, 16d aufweisen oder es kann eine Ausführung in 2-Komponentenspritztechnik oder das Umspritzen eines metallischen Einlegeteils oder eine ausgewählte Kombination dieser Alternativen gewählt werden. In dem gezeigten Beispiel ist die Wandstärke des Zwischenabschnitts 16e geringer als die Wandstärke des ersten Abschnitts 16c.

Am proximalen Ende der Antriebs- und Dosiervorrichtung ist ein als Betätigungsknopf ausgestaltetes Betätigungsglied 7 angeordnet, welches durch den Verwender der Vorrichtung für eine Produktausschüttung betätigbar, insbesondere in distale Richtung drückbar ist. Das Betätigungsglied 7 ist im Bezug auf das Dosisanzeigeelement 10 so angeordnet, dass es seine Position während der Dosiseinstellung hierzu nicht ändert. Das Betätigungsglied 7 ist insbesondere um einen Betätigungshub verschiebbar in dem Dosierglied 3 aufgenommen. Zwischen dem Dosierglied 3 und dem Betätigungsglied 7 wirkt eine Kupplungs- oder Rücksetzfeder 12, welche als Wendelfeder ausgestaltet ist und als Druckfeder wirkt. Die Feder 12 stützt sich an dem Dosierglied 3 und dem Betätigungsglied 7 ab. Das Betätigungsglied 7 bildet das proximale Ende der Antriebs- und Dosiervorrichtung.

Das Betätigungsglied 7 ist zumindest axialfest, vorzugsweise auch drehfest mit dem proximalen Ende des Kupplungsglieds 2 axialfest verbunden, insbesondere verschnappt. Das Betätigungsglied 7 ist zwischen einer betätigten und einer unbetätigten Position gegen die Druckkraft der Feder 12 insbesondere um einen Betätigungs- oder Kupplungshub hin und her verschiebbar.

Die Antriebs- und Dosiervorrichtung weist ein Vortriebsglied 8 in der Gestalt einer Kolbenstange auf, an deren distalem Ende ein Flansch 8c angeordnet bzw. befestigt ist. Das Vortriebsglied 8 wirkt auf den Kolben des Produktbehälters 14 oder stößt bevorzugt an dem Kolben an. Das Vortriebsglied 8 weist ein Außengewinde 8a auf, welches von einer Nut 8b überlagert ist, die sich in Längsrichtung des länglichen Vortriebsglieds 8 erstreckt.

Das Vortriebsglied 8 wird von einem Rotationsglied 1 umgeben, welches vorzugsweise hülsenförmig ist und drehfest und axial verschiebbar in Bezug auf das Vortriebsglied 8 ist.

Das Rotationsglied 1 weist einen Steg 1b auf, welcher für die rotationsfeste und axial verschiebbare Verbindung in die Nut 8b eingreift. Das Rotationsglied 1 weist an seinem Umfang ein nockenförmiges, an einem Federarm federnd angeordnetes Eingriffsglied 1c auf, welches in einem Eingriff mit einer gehäusefesten, insbesondere von dem Gehäuse 4 gebildeten Innenverzahnung 4c ist.

Das Außengewinde 8a des Vortriebsglieds 8 ist in einem Gewindeeingriff mit einem Innengewinde 13a einer Gewindehülse 13, so dass die Gewindehülse 13 an dem Vortriebsglied 8 entlang schraubbar ist. Die Gewindehülse 13 ist drehfest und axial verschiebbar mit dem Kupplungsglied 2 verbunden, so dass das Kupplungsglied 2 die Gewindehülse 13 mitdreht. Hierzu kann in der Gewindehülse 13 eine Innenhülse 13d gebildet sein, welche über mindestens einen, vorzugsweise zwei oder mehrere Stege 13e speichenartig mit dem Innenumfang der Gewindehülse 13 verbunden ist. Der mindestens eine Steg 13e greift durch je einen, vorzugsweise zwei oder mehrere axial sich erstreckende Führungsschlitze 2c der Kupplungshülse 2 und realisiert so die drehfeste Verbindung mit dieser und eine axiale Beweglichkeit vorzugsweise für den Betätigungs- oder Kupplungshub derselben.

Die Gewindehülse 13 ist an einem Lagerelement 9 drehbar und axialfest befestigt. Hierfür kann die Gewindehülse 13 zum Beispiel eine Ringnut 13c aufweisen, in welche das Lagerelement 9 zum Beispiel mit einem Ringsteg 9c eingreift. Das Lagerelement 9 ist im Bezug auf das Gehäuse 4 verdrehgesichert und axial verschiebbar. Das Lagerelement 9 greift für die verdrehfeste und verschiebbare Verbindung mit dem Gehäuse 4, insbesondere mit einer Abragung 9b in eine Nut 4d des Gehäuses 4, die sich in Längsrichtung erstreckt.

Das Lagerelement 9 weist ein Außengewinde 9a auf, welches in einem Gewindeeingriff mit einem Innengewinde 10b des als Dosisanzeigetrommel gebildeten Dosisanzeigeelements 10 steht. Somit ist das Dosisanzeigeelement 10 an dem Lagerelement 9 entlang schraubbar.

Das Dosisanzeigeelement 10 weist über seinen Umfang eine mehrfach umlaufende, wendelförmige oder helixförmige Dosisskala 10a auf, welche aus einer Vielzahl aneinander gereihten, insbesondere in internationalen Einheiten (IU) angegebenen Dosiswerten gebildet wird. Die Skala kann zum Beispiel in Einser- oder Zweierschritten die einstellbaren Dosiswerte 0 bis 60 oder 80 IU aufweisen. Durch Drehung des Dosierglieds 3 relativ zu dem Gehäuse 4 oder der Zeigeeinrichtung 4a kann die gewünschte auszuschüttende Produktdosis eingestellt werden, wobei der entsprechende Dosiswert an der Zeigeeinrichtung 4a ablesbar ist oder in der Zeigeeinrichtung 4a erscheint.

Das Dosisanzeigelement 10 ist insbesondere permanent drehfest und axial verschiebbar in Bezug auf das Kupplungsglied 2. Das Dosisanzeigelement 10 weist an seinem Innenumfang eine Abragung 10d auf, welche in eine sich in Längsrichtung erstreckende Nut 2d des Kupplungsglieds 2 eingreift.

Das Kupplungsglied 2 ist insbesondere mit einer zweiten Kupplung 2a, 1a mit dem Rotationsglied 1 verdrehgesichert verbindbar. Hierzu weist das Kupplungsglied 2 an seinem distalen Ende eine Kupplungsstruktur 2a in der Gestalt einer Innenverzahnung auf. Das Kupplungsglied 1b weist eine Kupplungsstruktur 1a in der Gestalt einer Außenverzahnung auf. Bei unbetätigtem Betätigungsglied 7 ist die zweite Kupplung 1a, 2a geöffnet und die erste Kupplung 2b, 16b geschlossen, so dass das Kupplungsglied 2 relativ zu dem Rotationsglied 1 und/oder zu dem Vortriebsglied 8 drehbar ist, wobei das Dosierglied 3 im Bezug auf das Rotationsglied 2 im Wesentlichen - abgesehen von einer gewissen Elastizität des Kupplungselements 16 - verdrehgesichert mit dem Kupplungsglied 2 verbunden ist. Ist das Betätigungsglied 7, insbesondere vollständig betätigt, ist die zweite Kupplung 1a, 2a geschlossen, wodurch das Kupplungsglied 2 relativ zu dem Rotationsglied 1 und/oder zu dem Vortriebsglied 8 verdrehgesichert ist, und die erste Kupplung 2b, 16b geöffnet, wodurch das Kupplungsglied 2 relativ zu dem Dosierglied 3 und/oder zu dem Gehäuse 4 drehbar ist. Zwischen der unbetätigten und der vollständig betätigten Position des Betätigungsglieds 7 gibt es eine Zwischenposition, bei der die zweite Kupplung 1a, 2a und die erste Kupplung 2b, 16b geschlossen sind. Hierdurch wird vorteilhaft verhindert, dass das Kupplungsglied 2 für eine Drehung relativ zu dem Gehäuse 4 bereits freigegeben ist, wenn die zweite Kupplung 1a, 2a noch nicht geschlossen ist. Dies würde nämlich zu einer Fehlfunktion der Antriebs- und Dosiervorrichtung führen.

Sobald die erste Kupplung 2b, 16b geöffnet ist, kann die vorgespannte Feder 11 das Kupplungsglied 2, die Gewindehülse 13 und, über die geschlossene zweite Kupplung 1a, 2a, das Rotationsglied 1 und das Vortriebsglied 8 relativ zu dem Gehäuse 4 verdrehen, wodurch das Vortriebsglied 8 in Ausschüttrichtung, das heißt in Richtung Kolben verschoben wird und die eingestellte Dosis ausschüttet aufgrund der relativen Drehung zwischen einem Gewinde 13b an der Gewindehülse 13 und einem gehäusefesten Gewinde 4b sowie keiner relativen Drehung zwischen dem Innengewinde 13a und dem Außengewinde 8a des Vorschubglieds 8.

Für die Dosiseinstellung, das heißt bei unbetätigtem Betätigungsglied 7, ist das Kupplungsglied 2 rotatorisch von dem Vortriebsglied 8 entkoppelt, so dass die Dosierbewegung keine Ausschüttbewegung des Vortriebsglieds 8 bewirkt. Die Steigung P₁ der Dosisskala 10a ist größer als die Steigung der Gewinde 10b, 9a. Damit sich das Dosisanzeigeelement 10 entsprechend der Steigung P₁ der Dosisskala 10a auf einer wendel- oder helixförmigen Bahn bewegt, welche die gleiche Steigung wie die Dosisskala 10a aufweist, wird das Lagerelement 9 von der Gewindehülse 13 um die Differenz zwischen der Steigung P₁ und der Steigung des Gewindes 10b verschoben. Hierzu kann die Gewindehülse 13 ein Gewinde 13a, 13b aufweisen, welches eine Steigung aufweist, die gleich der Differenz aus der Steigung P₁ und der Steigung des Gewindes 10b ist. Mit anderen Worten ergibt die Summe aus dem Gewinde 13a, 13b und dem Gewinde 9a die Steigung P₁ der Dosisskala 10a.

Die Gewindehülse 13 ist während der Dosiseinstellung relativ zu dem Vortriebsglied 8 drehbar und während der Dosisausschüttung relativ zu dem Vortriebsglied 8 nicht drehbar. Die Gewindehülse 13 weist zusätzlich zu dem Innengewinde 13a, welches in das Außengewinde 8a des Vortriebsglieds 8 eingreift, das Außengewinde 13b mit gleicher Gewindesteigung wie das Gewinde 13a auf. Das Außengewinde 13b greift in das gehäusefeste Gewinde 4b, welches von dem Gehäuse 4 gebildet wird, ein. Hierdurch wird bewirkt, dass sich die Gewindehülse 13 während der Dosiseinstellung relativ zu dem Gehäuse 4 um den gleichen Weg entlang der Längsachse L bewegt, wie es sich relativ zu dem Vortriebsglied 8 bewegt. Wegen der beiden Gewinde 13a, 13b braucht das Vortriebsglied 8 in keinem Eingriff mit dem Gehäuse 4 zu sein.

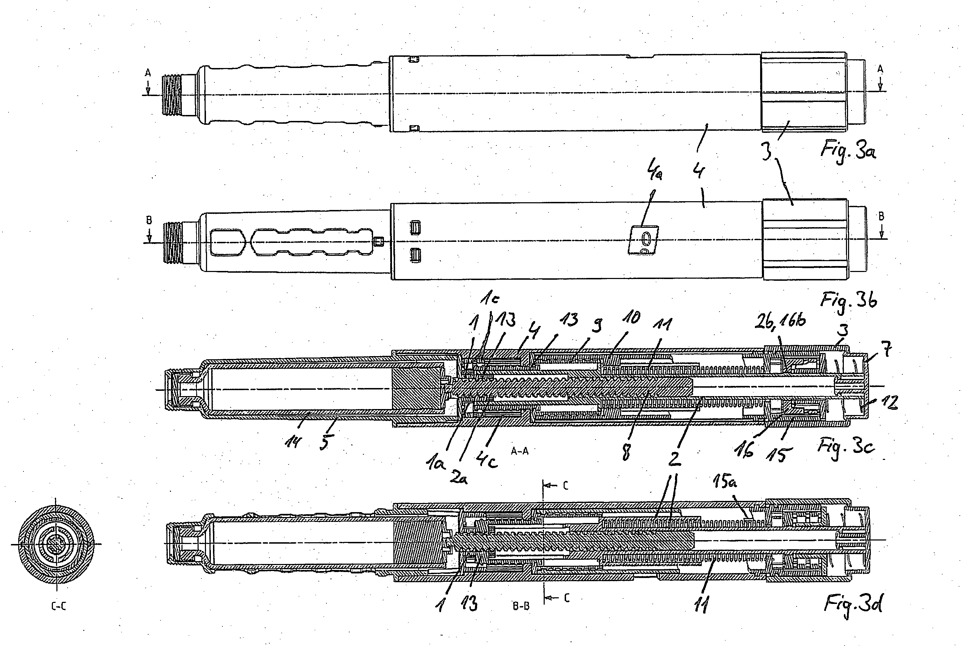

Bezugnehmend auf die Figuren 3a-d wird die Antriebs- und Dosiervorrichtung, die zusammen mit dem Produktbehälter 14 und dem Produktbehälterhalter 5 eine Injektionsvorrichtung bildet, in einer Ausgangsposition oder in einem Auslieferungszustand gezeigt, wobei die Dosis Null eingestellt ist. Das Betätigungsglied 7 ist unbetätigt, wobei die Feder 12 das Betätigungsglied 7 in proximale Richtung drückt, so dass die erste Kupplung 2b, 16b geschlossen und die zweite Kupplung 1a, 2a geöffnet ist. Die als Ausschüttfeder wirkende Drehfeder 11 ist im Auslieferungszustand vorzugsweise vorgespannt.

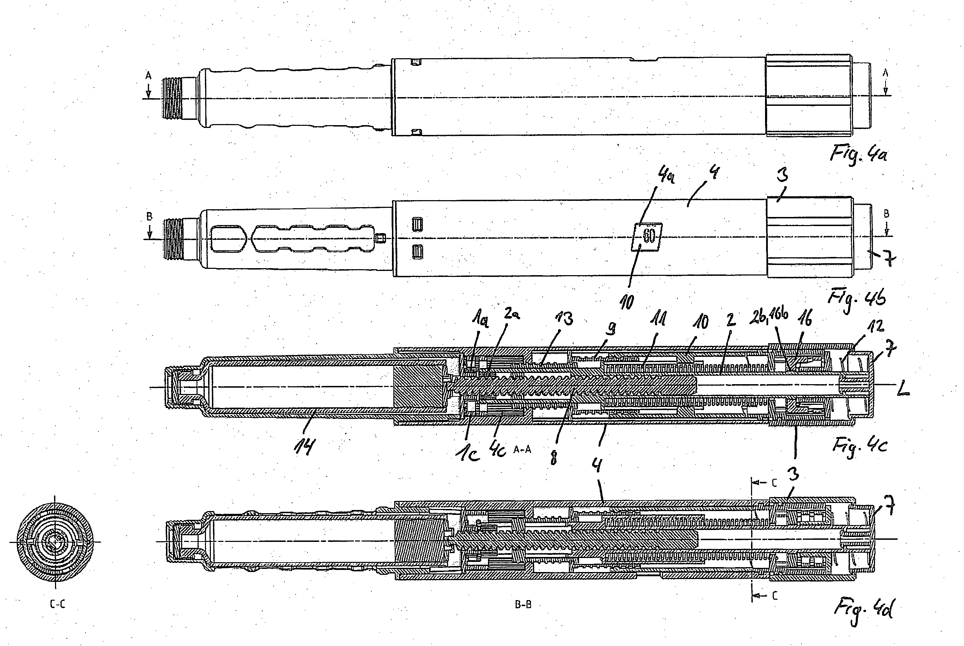

Mittels Drehung des Dosierglieds 3 in die zweite Drehrichtung wird über die geschlossene erste Kupplung 2b, 16b das Kupplungsglied 2 relativ zu dem Gehäuse 4 gedreht, wodurch sich die Gewindehülse 13 mit ihrem Außengewinde 13b an dem Gehäuse 4 und mit ihrem Innengewinde 13a an dem Vortriebsglied 8 entlangschraubt, insbesondere in proximale Richtung, wodurch es das Lagerelement 9 in Axialrichtung mitnimmt und sich das Lagerelement 9 verdrehgesichert entlang des Gehäuses 4 in proximale Richtung verschiebt. Aufgrund der Drehung des Kupplungsglieds 2 wird das Dosisanzeigeelement 10 mitgedreht und schraubt sich somit mit seinem Gewinde 10b an dem Lagerelement 9 entlang. Der Schraubbewegung des Dosisanzeigeelements 10 relativ zu dem Lagerelement 9 wird die Axialbewegung des Lagerelements 9 relativ zu dem Gehäuse 4 überlagert, wodurch das Dosisanzeigeelement 10 in Bezug auf das Gehäuse 4 eine wendel- oder helixförmige Bahn beschriebt, die der Steigung P₁ der Dosisskala 10a entspricht. Bei der Dosiserhöhung wird der Maximaldosisanschlag 10c zu dem Maximaldosisgegenanschlag 15a hinbewegt. Ist die mit der Antriebs- und Dosiervorrichtung maximal einstellbare Dosis, wie hier beispielsweise mit 60 IU angegeben, erreicht, schlägt der Maximaldosisanschlag 10c an den Maximaldosisgegenanschlag 15a an (Figuren 4a-d). Durch Drehung des Dosierglieds 3 in die erste Drehrichtung kann die eingestellte Dosis korrigiert oder verringert werden, wobei sich der Maximaldosisanschlag 10c von dem Maximaldosisgegenanschlag 15a wegbewegt und sich ein Nulldosisanschlag 10e, der durch die Stirnseite des Dosisanzeigeelements 10 gebildet wird und als Axialanschlag wirkt zu einem Nulldosisgegenanschlag 9e, der von einem Kragen des Lagerelements 9 gebildet wird, hinbewegt.

Bei der Drehung des Dosierglieds 3 in die erste Drehrichtung wird die Feder 11 entspannt, wobei sie bei der Drehung in die zweite Drehrichtung gespannt wird. Lässt der Verwender das Dosierglied 3 los, verhindert das Kupplungselement 16, dass sich die Feder 11 entspannt.

Zum Ausschütten der eingestellten Dosis wird das Betätigungsglied 7 gegen die Kraft der Feder 12 in distale Richtung verschoben, wodurch die zweite Kupplung 1a, 2a geschlossen und die erste Kupplung 2b, 16b geöffnet wird. Die Feder 11 treibt nun das Kupplungsglied 2 in die erste Drehrichtung rotatorisch an, wobei das Vortriebsglied 8 relativ zu der Gewindehülse 13 still steht und sich zusammen mit der Gewindehülse 13 mittels des Gewindes 13b und des Gewindes 4b an dem Gehäuse 4 in distale Richtung schraubt und somit den Kolben in den Produktbehälter 14 verschiebt. Hierbei wird das Eingriffsglied 1c über die Verzahnung 4c bewegt, wodurch die Ausschüttung der Produktdosis mittels Klickgeräuschen signalisiert wird. Das Eingriffsglied 1c bildet mit der Verzahnung 4c ferner eine unidirektionale Kupplung, die bewirkt, dass das Rotationsglied 1 nur in eine Richtung, nämlich in die erste Drehrichtung drehbar ist, welche eine Produktausschüttung bewirkt.

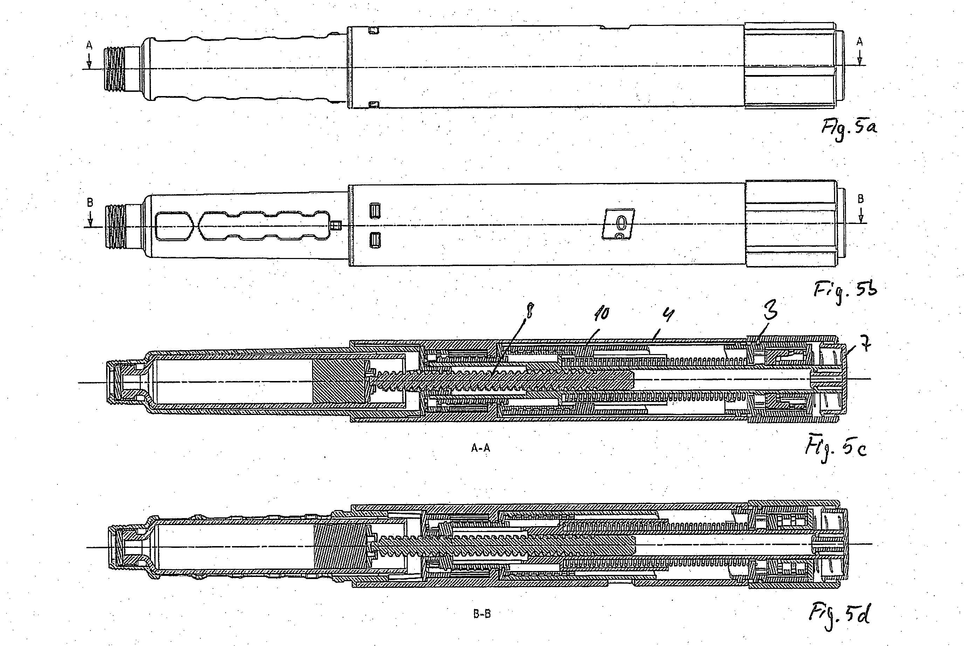

Durch die Drehbewegung des Kupplungsglieds 2 in die erste Drehrichtung wird das Dosisanzeigeelement 10 an dem Lagerelement 9 zurückgeschraubt, insbesondere der Nulldosisanschlag 10e in Richtung Nulldosisgegenanschlag 9e bewegt, wodurch die Dosisskala 10a in dem Zeigeelement 4 zur Nulldosis hin zurück zählt. Wenn die Dosis Null in der Zeigeeinrichtung 4a angezeigt wird (Figuren 5a-d) oder der Nulldosisanschlag 10e an dem Nulldosisgegenanschlag 9e anschlägt, ist die Ausschüttung beendet. In den Figuren 5a, 5b wird die Antriebs- und Dosiervorrichtung am Ende der Produktausschüttung gezeigt, wobei das Betätigungsglied 7 noch betätigt ist, d. h. vom Verwender der Vorrichtung noch nicht losgelassen wurde.

Durch wiederholtes Dosieren und Betätigen des Betätigungsglieds 7 kann das in dem Produktbehälter 14 enthaltene Produkt in mehreren beliebig wählbaren Einzeldosen ausgeschüttet werden. Auch kann durch Loslassen bzw. wieder Betätigen des Betätigungsglieds eine Ausschüttung unterbrochen bzw. wieder aufgenommen werden.

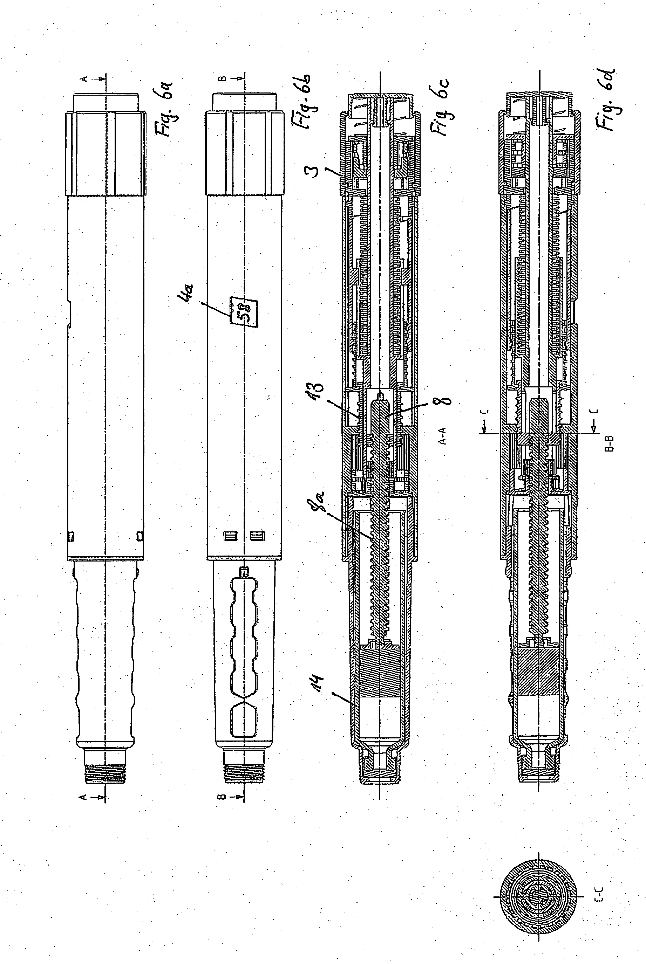

In den Figuren 6a-d wird der Zustand der Antriebs- und Dosiervorrichtung dargestellt, bei der in dem Produktbehälter 14 eine ausschüttbare Dosis des Produkts enthalten ist, die geringer als die mit der Antriebs- und Dosiervorrichtung einstellbare Maximaldosis ist. In dem gezeigten Beispiel sind nur noch 58 IU in dem Produktbehälter 14 enthalten, wobei mit der Antriebs- und Dosiervorrichtung maximal 60 IU einstellbar wären. Um eine Fehlanwendung zu vermeiden, umfasst die Antriebs- und Dosiervorrichtung eine Begrenzungseinrichtung, welche die Dosiseinstellung begrenzt. Hierzu weist das Vortriebsglied 8 einen Anschlag am proximalen Ende des Gewindes 8a auf, an dem die Gewindehülse 13 anschlägt, wodurch eine Drehung des Dosierglieds 2 in die zweite Drehrichtung blockiert wird, auch wenn der Maximaldosisanschlag 10c nicht in dem Anschlag mit dem Maximaldosisgegenanschlag 15a ist. Eine Drehung des Dosierglieds 3 in die erste Drehrichtung ist jedoch möglich.

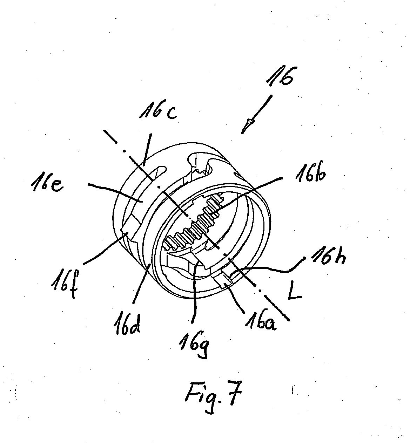

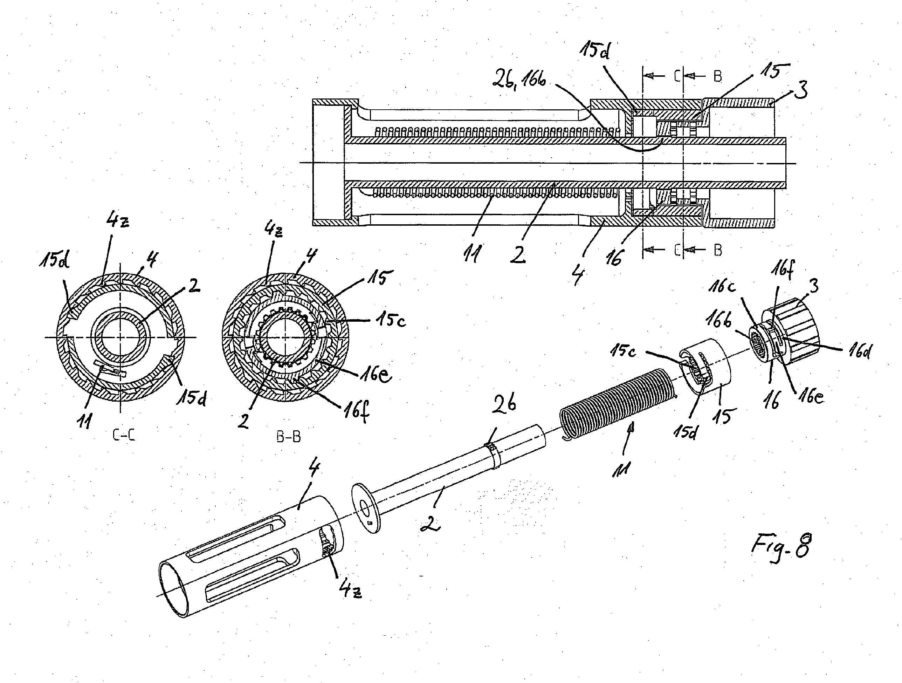

In Figur 8 wird eine Sperreinrichtung gezeigt, dessen Kupplungselement 16 dem der Vorrichtung aus den Figuren 1 bis 6d sehr ähnlich ist und sich vor allem durch die Form des Eingriffsglieds 4f und dadurch unterscheidet, dass das Aufziehglied 3 integral, d.h. einteilig, an einem zweiten Abschnitt des Kupplungselements 16 gebildet ist. Insbesondere hinsichtlich der Ausgestaltung des Kupplungselements 16 und der Funktion des Lösens des Eingriffsglieds 4f aus dem Eingriff mit der Verzahnung 15c wird auf die Beschreibung zu den Figuren 1 bis 7 verwiesen.

Die Sperranordnung aus Figur 8 umfasst ein zwischen dem Aufziehglied 3 und dem Drehglied 2 angeordnetes Kupplungselement 16. Durch Drehung des Aufziehglieds 3 relativ zu einem hülsenförmigen Gehäuse 4 in eine erste Drehrichtung ist die wendelförmige, als Drehfeder wirkende Feder 11 entspannbar und in eine zweite, der ersten Drehrichtung entgegengesetzte Drehrichtung spannbar.

Ein Entspannen der Feder 11 wird insbesondere durch das Kupplungselement 16 verhindert, wenn das Aufziehglied 3 unbelastet ist, d.h. frei von äußeren, wie z.B. vom Verwender aufgebrachten Drehmomenten ist.

Das Eingriffsglied 16f weist eine in die erste Drehrichtung weisende, erste Zahnflanke und eine in die zweite Drehrichtung weisende, zweite Zahnflanke auf. Die ersten und zweiten Zahnflanken sind in dem gezeigten Beispiel in etwa gleich steil angeordnet, so dass das Eingriffsglied 16f trapezförmig ist.

Die Verzahnung 15c weist eine Vielzahl über den Umfang verteilte Zähne auf. Ein Zahn oder mehrere dieser Zähne, insbesondere jeder dieser Zähne, können zum Beispiel sägezahnförmig oder trapezförmig gebildet sein. Sie können eine in Umfangsrichtung weisende erste Flanke und eine entgegengesetzt zu der ersten Flanke in Umfangsrichtung weisende zweite Flanke aufweisen, wobei die erste Flanke steiler als die zweite Flanke oder in etwa gleich steil wie die zweite Flanke ausgebildet ist. Die erste Flanke bildet eine Gegenflanke für die erste Flanke des Eingriffsglieds 16f.

Der Eingriff zwischen dem Eingriffsglied 16f und der Verzahnung ist so, dass das Kupplungselement 16 in Bezug auf die Hülse 15 in die zweite Drehrichtung nicht drehbar, d.h. drehfest ist.

Die Feder 11 stützt sich mit einem, insbesondere dem distalen Ende, an einem relativ zu dem Gehäuse 4 drehbaren Drehglied 2 und mit dem anderen, insbesondere dem proximalen Ende, gehäusefest, insbesondere an dem Gehäuseeinsatz 15 ab. Eine Drehung des Drehglieds 2 in die zweite Drehrichtung bewirkt ein Spannen der Drehfeder 11, wobei eine Drehung in die erste Drehrichtung ein Einspannen der Feder 11 bewirkt. Das Drehglied 2 ist vorzugsweise über eine lösbare Kupplung 2b, 16b im Wesentlichen verdrehgesichert mit dem Aufziehglied 3 verbunden. Das Kupplungsglied 2 weist zur Bildung der Kupplung 2b, 16b eine Kupplungsstruktur 2b auf. Der erste Abschnitt 16c des Kupplungselements 16 weist zur Bildung der zweiten Kupplung 2b, 16b eine Kupplungsstruktur 16b auf, welche bei geschlossener Kupplung 2b, 16b in einem verdrehgesicherten Eingriff mit der Kupplungsstruktur 2b ist.

Das Aufziehglied 3 ist mit dem zweiten Abschnitt 16d des Kupplungselements 16 einstückig gebildet, wie z.B. ein aus Kunststoff gebildetes Spritzgrussteil.

Die Verzahnung 15c wird an dem Innenumfang einer ersten Hülse 15, insbesondere Ratschenhülse, gebildet, welche relativ zu dem Gehäuse 4 nur in die zweite Drehrichtung drehbar ist und in die erste Drehrichtung verdrehgesichert ist. Die erste Hülse 15 weist ein federnd angeordnetes Eingriffsglied 15d auf, welches in solch einem Eingriff mit einer Verzahnung 4z einer zweiten Hülse, welche von dem hülsenförmigen Gehäuses 4 gebildet wird, steht, dass die erste Hülse 15 in die zweite Drehrichtung drehbar ist und in die erste Drehrichtung nicht drehbar ist. Diese Anordnung kann auch als Ratsche bezeichnet werden. Die Zähne der Verzahnung 4z sind sägezahnförmig, d.h. mit einer flachen und einer steilen Flanke, wobei das bevorzugt sägezahnförmige Eingriffsglied 15d über die flache Flanke abgleiten kann und an die steile Flanke anschlägt.

Eine Drehung des Aufziehglieds 3 oder/und des Kupplungselements 16 in die zweite Drehrichtung bewirkt, dass sich die erste Hülse 15 mit dem Kupplungselement 16 mitdreht, wobei das Eingriffsglied 15d der Hülse 15 über die Verzahnung 4z des Gehäuses 4 rastet. Durch die Drehung in die zweite Drehrichtung wird die Feder 11 gespannt.

Das Drehglied 2 ist relativ zu dem Kupplungselement 16 axial verschiebbar, wodurch die erste Kupplung 2b, 16b, die im geschlossenen Zustand den zweiten Abschnitt des Kupplungselements 16 verdrehgesichert mit dem Drehglied 2 verbindet, geöffnet wird, so dass das Drehglied 2 relativ zu dem Kupplungselement 16 in die erste Drehrichtung drehbar ist, wodurch die Feder 11 die in ihr gespeicherte Federenergie an das Drehglied 2 abgeben kann.

Mit dem Drehglied 2 kann z.B. eine Kolbenstange einer Injektionsvorrichtung so, insbesondere über eine zweite Kupplung (Figuren 1 bis 6d) gekoppelt sein, dass die Drehung des Drehglieds 2 eine Vortriebsbewegung der Kolbenstange bewirkt. Hierdurch kann ein Produkt aus einem Produktbehälter ausgeschüttet werden.

**Bezugszeichenliste**

| | | | |
|---|---|---|---|
| 1 | Rotationsglied | 10a | Dosisskala |
| 1a | zweite Kupplungsstruktur | 10b | Gewinde / Innengewinde |
| 1b | Steg | 10c | Maximaldosisanschlag |
| 1c | Eingriffsglied / Nocken | 10d | Eingriffsnocken / Abragung |
| | | 10e | Nulldosisanschlag |
| 2 | Kupplungsglied/erstes Drehglied | | |
| 2a | erste Kupplungsstruktur/ Innenverzahnung | 11 | Feder / Ausschüttfeder / Drehfeder |
| 2b | dritte Kupplungsstruktur | 12 | Kupplungsfeder / Rücksetzfeder |
| 2c | Führungsschlitz | | |
| 2d | Führung Längsführung/ Nut | 13 | Gewindehülse |
| | | 13a | Innengewinde / Gewinde |
| 3 | Dosierglied/ Aufziehglied/zweites Drehglied | 13b | Außengewinde / Gewinde |
| | | 13c | Ringnut |
| 3a | Abragung Längssteg | 13d | Innenhülse |
| 3b | Ringsteg | 13e | Steg |
| 4 | Gehäuse | 14 | Produktbehälter |
| 4a | Zeigeeinrichtung | | |
| 4b | Gewinde / Innengewinde | 15 | Gehäuseeinsatz / erste Hülse / Ratschenhülse |
| 4c | Verzahnung / Innenverzahnung | | |
| 4d | Längsführung / Nut | 15a | Maximaldosisgegenanschlag |
| 4e | Innengewinde | 15b | Ringnut |
| 4f | Eingriffsglied | 15c | Innenverzahnung / Verzahnung |
| 4z | Verzahnung | 15d | Eingriffsglied |
| 5 | Produktbehälter | 16 | Kupplungselement / 2-Wege-Kupplung |
| 6 | Kappe (nicht gezeigt) | 16a | Anschlag / Kante / Nut |
| | | 16b | Verdrehsicherung / vierte Kupplungsstruktur |
| 7 | Betätigungsglied / Betätigungsknopf | | |
| | | 16c | erster Abschnitt |
| | | 16d | zweiter Abschnitt |
| 8 | Vortriebsglied / Stößel / Kolbenstange | 16e | Zwischenabschnitt |
| | | 16f | Eingriffsglied / erste Zahnflanke / zweite Zahnflanke |
| 8a | Außengewinde | | |
| 8b | Längsführung / Nut | 16g | zweiter Anschlag / Kante |
| 8c | Flansch | 16h | erster Anschlag / Nutflanke |
| 9 | Lagerelement | L | Längsachse / Drehachse |
| 9a | Gewinde/Außengewinde | P₁ | Steigung der Dosisskala |
| 9b | Abragung, | | |
| 9c | Ringsteg | | |
| 9e | Nulldosisgegenanschlag | | |
| 10 | Dosisanzeigeelement | | |

## Patentansprüche

1. Sperreinrichtung, umfassend:
a) eine Hülse (15), welche über ihren Innenumfang eine Verzahnung (15c) aufweist,
b) ein hülsenförmiges, insbesondere einstückiges Kupplungselement (16) mit einer Längsachse (L), das über seinen Umfang von der Hülse (15) umgeben ist und das einen ersten Abschnitt (16c) und einen zweiten Abschnitt (16d) aufweist, wobei der erste Abschnitt (16c) mit dem zweiten Abschnitt (16d) über einen elastisch verformbaren Zwischenabschnitt (16e) verbunden ist, wobei der Zwischenabschnitt (16e) ein Eingriffsglied (16f) aufweist, welches in die Verzahnung (15c) der Hülse (15) eingreift,
c) ein Drehmomentbeaufschlagungsmittel (11), insbesondere eine Dreh- oder Ausschüttfeder, welches den ersten Abschnitt (16c) des Kupplungselements (16) um die Längsachse (L) mit einem in eine erste Drehrichtung gerichteten Drehmoment beaufschlagt, wobei das Eingriffsglied (16f) durch das Drehmoment in die Verzahnung (15c) gedrückt wird, so dass eine Drehung des Kupplungselements (16) relativ zu der Hülse (15) in die erste Drehrichtung blockiert wird,
d) wobei der zweite Abschnitt (16d) des Kupplungselements (16) relativ zu dem ersten Abschnitt (16c) um die Längsachse (L) in die erste Drehrichtung tordierbar ist, wodurch der die Verdrehung in die erste Drehrichtung sichernde Eingriff des Eingriffsglieds (16f) in die Verzahnung (15c) lösbar ist, so dass eine Drehung des Kupplungselements (16) relativ zu der Hülse (15) in die erste Drehrichtung freigegeben ist.

2. Sperreinrichtung nach dem vorhergehenden Anspruch, **dadurch gekennzeichnet, dass** das Kupplungselement (16) relativ zu der Hülse (15) in eine zweite, der ersten Drehrichtung entgegengesetzte Drehrichtung drehbar ist, wobei das Eingriffsglied (16f) über die Verzahnung rastet.

3. Sperreinrichtung nach Anspruch 1, **dadurch gekennzeichnet, dass** das Kupplungselement (16) relativ zu der Hülse (15) in die zweite, der ersten Drehrichtung entgegengesetzte Drehrichtung drehfest ist, wobei die Hülse (15) ein weiteres elastisch angeordnetes Eingriffsglied (15d) aufweist, welches in eine Verzahnung (4z) einer die Hülse (15) umgebenden Hülse (4) eingreift, wobei das weitere Eingriffsglied (15d) über die Verzahnung (4z) der die Hülse (15) umgebenden Hülse (4) rastet, wodurch das Kupplungselement (16) in die zweite Drehrichtung drehbar ist.

4. Sperreinrichtung nach einem der vorhergehenden Ansprüche, **dadurch gekennzeichnet, dass** die Verzahnung (4z; 15c) eine Vielzahl Zähne aufweist, wobei ein oder mehrere dieser Zähne eine in eine Umfangsrichtung weisende erste Flanke und eine entgegengesetzt zu der ersten Flanke in Umfangsrichtung weisende zweite Flanke aufweisen, wobei z.B. die die erste Flanke steiler und als die zweite Flanke ausgebildet ist.

5. Sperreinrichtung nach einem der vorhergehenden Ansprüche, **dadurch gekennzeichnet, dass** das Eingriffsglied (16f) zahnförmig ist, wobei das Eingriffsglied (16f) eine in die erste Drehrichtung weisende erste Flanke und eine in die zweite Drehrichtung weisende zweite Flanke aufweist, wobei die erste Flanke steiler und als die zweite Flanke ausgebildet ist.

6. Sperreinrichtung nach einem der vorhergehenden Ansprüche, **dadurch gekennzeichnet, dass** der Zwischenabschnitt (16e) mit einem Ende in den ersten Abschnitt (16c) und mit dem anderen Ende in den zweiten Abschnitt (16d) mündet, wobei das Eingriffsglied (16f) zwischen den beiden Enden angeordnet ist.

7. Sperreinrichtung nach einem der vorhergehenden Ansprüche, **dadurch gekennzeichnet, dass** sich der Zwischenabschnitt (16e) von dem ersten Abschnitt (16c) in die erste Drehrichtung erstreckt und/oder von dem zweiten Abschnitt (16d) entgegen die erste Drehrichtung erstreckt.

8. Sperreinrichtung nach einem der vorhergehenden Ansprüche, **dadurch gekennzeichnet, dass** ein zweites Drehglied (3) so mit dem Kupplungselement (16) verbunden ist, dass es gegen einen ersten Drehanschlag (16h) des Kupplungselements (16) gedrückt wird, wenn es in die erste Drehrichtung gedreht wird, und gegen einen in die entgegengesetzte Drehrichtung wirkenden zweiten Drehanschlag (16g) gedrückt wird, wenn es in die zweite Drehrichtung gedreht wird, wobei der erste Drehanschlag (16h) an dem zweiten Abschnitt (16d) und der zweite Drehanschlag (16g) an dem ersten Abschnitt (16c) oder dem Zwischenabschnitt (16e) gebildet ist.

9. Sperreinrichtung nach einem der vorhergehenden Ansprüche, **dadurch gekennzeichnet, dass** der erste Abschnitt (16c) und/oder der zweite Abschnitt (16d) eine Verdrehsicherungsstruktur (16a, 16b) aufweist.

10. Antriebs- und/oder Dosiervorrichtung für eine Injektionsvorrichtung zur Verabreichung eines flüssigen Produkts, insbesondere eines Medikaments, umfassend:
- ein insbesondere vom Verwender der Vorrichtung greifbares zweites Drehglied (3), insbesondere Aufziehglied, und eine mittels Drehung des zweiten Drehglieds (3) spannbare Feder (11), wobei die Feder (11) die für die Produktausschüttung erforderliche Energie speichert,
**gekennzeichnet durch**
- eine Sperreinrichtung nach einem der vorhergehenden Ansprüche, wobei die Sperreinrichtung zwischen, insbesondere kinematisch zwischen dem zweiten Drehglied (3) und der Feder (11) angeordnet ist.

11. Antriebs- und/oder Dosiervorrichtung nach dem vorhergehenden Anspruch, **dadurch gekennzeichnet, dass** mit der Antriebs- und/oder Dosiervorrichtung mittels Drehung des zweiten Drehglieds (3) eine zu verabreichende Produktdosis einstellbar ist, ferner umfassend ein Dosisanzeigeelement (10), welches so mit dem zweiten Drehglied (3) gekoppelt ist, dass eine Drehung des zweiten Drehglieds (3) eine Drehung des Dosisanzeigeelements (10) bewirkt.

12. Antriebs- und/oder Dosiervorrichtung nach einem der zwei vorhergehenden Ansprüche, **gekennzeichnet durch** eine erste Kupplung (2b, 16b), welche zwischen, insbesondere kinematisch zwischen dem Kupplungselement (16) und der Ausschüttfeder (11) angeordnet ist, und **durch** ein für die Produktausschüttung betätigbares Betätigungsglied (7), wobei die erste Kupplung (2b, 16b) bei unbetätigtem Betätigungsglied (7) geschlossen und bei betätigtem Betätigungsglied (7) geöffnet ist oder **durch** Betätigung des Betätigungsglieds (7) geöffnet wird.

13. Antriebs- und/oder Dosiervorrichtung, nach dem vorhergehenden Anspruch, **dadurch gekennzeichnet, dass** die geschlossene erste Kupplung (2b, 16b) ein zwischen der Ausschüttfeder (11) und dem Kupplungselement (16) angeordnetes erstes Drehglied (2), insbesondere Kupplungsglied, drehfest mit dem Kupplungselement (16) verbindet, wobei das erste Drehglied (2) bei geschlossener erster Kupplung (2b, 16b) relativ zu einem auf einen Kolben eines Produktbehälters (14) wirkenden Vortriebsglied (8), insbesondere Kolbenstange, drehbar ist.

14. Antriebs- und/oder Dosiervorrichtung nach einem der vier vorhergehenden Ansprüche, **gekennzeichnet durch** ein längliches Vortriebsglied (8), dessen distales Ende insbesondere auf einen Kolben eines Produktbehälters (14) wirken kann, und dessen Verschiebung in distale Richtung bewirkt, dass der Kolben in dem Produktbehälter (14) verschoben wird, wobei das Vortriebsglied (8) so mit einem zwischen dem Kupplungselement (16) und der Antriebsfeder (11) angeordneten ersten Drehglied (2) verbunden oder verbindbar ist, dass eine Drehung des ersten Drehglieds (2) eine Bewegung des Vortriebsglieds (8) in distale Richtung bewirkt.

15. Antriebs- und/oder Dosiervorrichtung nach dem vorhergehenden Anspruch, **gekennzeichnet durch** ein für die Produktausschüttung betätigbares Betätigungsglied (7) und eine zweite Kupplung (1a, 2a), welche bei unbetätigtem Betätigungsglied (7) geöffnet und bei betätigtem Betätigungsglied (7) geschlossen ist oder **durch** Betätigung des Betätigungsglieds (7) geschlossen wird, wobei die geschlossene zweite Kupplung (1a, 2a) das erste Drehglied (2) und das Vortriebsglied (8) so miteinander verbindet, dass eine Drehung des ersten Drehglieds (2) die Bewegung des Vortriebsglieds (8) in distale Richtung bewirkt.

## Claims

1. Locking device, comprising
a) a sleeve (15) which has gear teeth (15c) around its inner circumference,
b) a sleeve-shaped, in particular one-piece coupling element (16) with a longitudinal axis (L) which is encircled around its circumference by the sleeve (15) and which has a first section (16c) and a second section (16d), wherein the first section (16c) is connected to the second section (16d) by means of an flexibly deformable intermediate section (16e) wherein the intermediate section (16e) has an engagement member (16f) which engages in the gear teeth (15c) of the sleeve (15),
c) a torque application means (11), in particular a torsion or discharge spring, which applies a torque directed in a first rotational direction around the longitudinal axis (L) to the first section (16c) of the coupling element (16), whereby the engagement element (16f) is pressed by the torque into the gear teeth (15c) so that the coupling element (16) is prevented from turning in the first rotational direction relative to the sleeve (15),
d) wherein the second section (16d) of the coupling element (16) can be twisted relative to the first section (16c) in the first rotational directionaround the longitudinal axis (L), by means of which the engagement of the engagement member (16f) in the gear teeth (15c) locking the rotation in the first rotational direction can be released, so that the coupling element (16) is now free to rotate in the first rotational direction relative to the sleeve (15).

2. Locking device in accordance with the previous claim, **characterised in that** the coupling element (16) can be rotated relative to the sleeve (15) in a second rotational direction opposite the first rotational direction, whereby the engagement member (16f) latches by means of the gear teeth.

3. Locking device in accordance with claim 1, characterised in thatthat the coupling element (16) is locked from turning relative to the sleeve (15) in the second rotational direction opposite the first rotational direction, wherein the sleeve (15) has a further flexibly arranged engagement member (15d) which engages in gear teeth (4z) of a housing (4) surrounding sleeve (15), whereby the furtherengagement member (15d) latches the housing (4) surrounding sleeve (15)by means of the gear teeth (4z), whereby the coupling element (16) can rotate in the second rotational direction.

4. Locking device in accordance with one of the previous claims,
**characterised in that** the gear teeth (4z; 15c) comprise a multiplicity of teeth wherein one or more of these teeth has/have a first flank pointing in a circumferential direction and a second flank opposite to the first flank pointing in a circumferential direction, wherein, for example, the first flank is formed steeper than the second flank.

5. Locking devicein accordance with one of the previous claims,
characterised in thatthe engagement member (16f) is tooth-shaped, wherein the engagement member (16f) has a first flank pointing in the first rotational direction and a second flank pointing in the second rotational direction wherein the first flank is formed steeper than the second flank.

6. Locking devicein accordance with one of the previous claims,
characterised in thatthe intermediate section (16e) leads, with one end, into the first section (16c) and, with the other end, into the second section (16d), wherein the engagement member (16f) is arranged between the two ends.

7. Locking devicein accordance with one of the previous claimscharacterised in thatthe intermediate section (16e) extends from the first section (16c) in the first rotational direction and/or extends from the second section (16d) opposite to the first rotational direction.

8. Locking devicein accordance with one of the previous claims, characterised in thata second rotary member (3) is connected with the coupling element (16) such that it is pressed against a first rotational stop (16h) of the coupling element (16) when it is rotated in the first rotational direction and is pressed against a second rotational stop (16g) acting in the opposite rotational direction when it is rotated in the second rotational direction, wherein the first rotational stop (16h) is formed on the second section (16d) and the second rotational stop (16g) is formed on the first section (16c) or on the intermediate section (16e).

9. Locking devicein accordance with one of the previous claims, **characterised in that** the first section (16c) and/or the second section (16d) have/has an anti-turn structure (16a, 16b).

10. Actuation and/or dosing device for an injection device for administering a liquid product, in particular a medication, comprising:
- a second rotary member (3), in particular a filling member, graspable in particular by the user of the device, and a spring (11) that can be tensioned by rotating the second rotary member (3), whereby the spring (11) stores energy needed for the discharge of the product,
**characterised by**
- a locking device in accordance with one of the previous claims, wherein the locking device is arranged between, in particular kinematically between the second rotary member (3) and the spring (11).

11. Actuation and/or dosing device in accordance with the previous claim, **characterised in that** a product dose to be administered can be adjusted with theactuation and/or dosing device by rotating the second rotary member (3), further comprising a dose indicator element (10) which is coupled with the second rotary member (3) such that a rotation of the second rotary member (3) causes the dose indicator element (10) to rotate.

12. Actuation and/or dosing device in accordance with one of the two previous claims, characterised bya first coupling (2b, 16b) which is arranged between, particularly kinematically between the coupling element (16) and the discharge spring (11), and by an actuator (7) activated for discharging the product, wherein the first coupling (2b, 16b) is closed with an inactivated actuator (7) and is openedwith, an activated actuator (7) or is opened by activating the actuator (7).

13. Actuation and/or dosing device in accordance with the previous claim, **characterised in that** the closed first coupling (2b, 16b) connects, so that it cannot rotate, a first rotary member (2), in particular a coupling element, arranged between the discharge spring (11) and the coupling element (16), with the coupling element (16), wherein the first rotary member (2) is able to rotate, with the first coupling (2b, 16b) closed, relative to a propulsion member (8), in particular a piston rod, acting on a piston of a product container (14).

14. Actuation and/or dosing device in accordance with one of the four previous claims, **characterised by** a longitudinal propulsion member (8) whose far end can act in particular on a piston of a product container (14), and whose movement in the direction of the far end results in pushing the piston in the product container (14), wherein the propulsion member (8) is connected or can be connected to a first rotary member (2) arranged between the coupling element (16) and the discharge spring (11) in such a way that a rotation of the first rotary member (2) causes the propulsion member (8) to move towards the far end.

15. Actuation and/or dosing device in accordance with the previous claim, **characterised by** an actuator (7) activated for discharging the product and a second coupling (1a, 2a) whichis opened with an inactivated actuator (7) and is closed with an activated actuator (7) or is closed by activating the actuator (7), wherein the closed second coupling (1a, 2a) connects the first rotary member (2) and the propulsion member (8) in such a way that a rotation of the first rotating element (2) causes thepropulsion member (8) to move towards the far end.

## Revendications

1. Dispositif d'arrêt, comportant :
a) une douille (15) qui comporte une denture (15c) sur son contour intérieur,
b) un élément de couplage (16) en forme de douille, en particulier d'un seul tenant, avec un axe longitudinal (L), qui est entouré sur sa périphérie par la douille (15) et qui comporte une première partie (16c) et une deuxième partie (16d), ladite première partie (16c) étant reliée à ladite deuxième partie (16d) par une partie intermédiaire (16e) élastiquement déformable, ladite partie intermédiaire (16e) comportant un organe d'engrènement (16f), qui engrène dans la denture (15c) de la douille (15),
c) un moyen d'application d'un couple de rotation (11), en particulier un ressort rotatif ou ressort de distribution, qui applique sur la première partie (16c) de l'élément de couplage (16) un couple de rotation dirigé dans un premier sens de rotation autour de l'axe longitudinal (L), ledit organe d'engrènement (16f) étant poussé dans la denture (15c) sous l'effet du couple de rotation, de manière à empêcher une rotation de l'élément de couplage (16) par rapport à la douille (15) dans le premier sens de rotation,
d) ladite deuxième partie (16d) de l'élément de couplage (16) pouvant subir une torsion dans le premier sens de rotation autour de l'axe longitudinal (L) par rapport à la première partie (16c), moyennant quoi l'engrènement de l'organe d'engrènement (16f) dans la denture (15c), lequel empêche la rotation dans le premier sens de rotation, peut être désolidarisé de manière à permettre une rotation de l'élément de couplage (16) par rapport à la douille (15) dans le premier sens de rotation.

2. Dispositif d'arrêt selon la revendication précédente, **caractérisé en ce que** l'élément de couplage (16) est apte à tourner par rapport à la douille (15) dans un deuxième sens de rotation, opposé au premier sens de rotation, l'organe d'engrènement (16f) s'arrêtant au-dessus de la denture.

3. Dispositif d'arrêt selon la revendication 1, **caractérisé en ce que** l'élément de couplage (16) est immobile en rotation par rapport à la douille (15) dans le deuxième sens de rotation opposé au premier sens de rotation, ladite douille (15) comportant un organe d'engrènement (15d) supplémentaire, agencé élastiquement et engrenant dans une denture (4z) d'une douille (4) entourant la douille (15), ledit organe d'engrènement (15d) supplémentaire s'arrêtant au-dessus de la denture (4z) de la douille (4) entourant la douille (15), moyennant quoi l'élément de couplage (16) est apte à tourner dans le deuxième sens de rotation.

4. Dispositif d'arrêt selon l'une quelconque des revendications précédentes, **caractérisé en ce que** la denture (4z ; 15c) comporte une pluralité de dents, une ou plusieurs de ces dents comportant un premier flanc orienté dans le sens circonférentiel et un deuxième flanc orienté dans le sens circonférentiel dans le sens opposé au premier flanc, le premier flanc ayant, par exemple, une plus forte pente que le deuxième flanc.

5. Dispositif d'arrêt selon l'une quelconque des revendications précédentes, **caractérisé en ce que** l'organe d'engrènement (16f) est réalisé en forme de dent, ledit organe d'engrènement (16f) comportant un premier flanc orienté dans le premier sens de rotation et un deuxième flanc orienté dans le deuxième sens de rotation, le premier flanc ayant une plus forte pente que le deuxième flanc.

6. Dispositif d'arrêt selon l'une quelconque des revendications précédentes, **caractérisé en ce que** la partie intermédiaire (16e) débouche avec une extrémité dans la première partie (16c) et débouche avec l'autre extrémité dans la deuxième partie (16d), l'organe d'engrènement (16f) étant disposé entre les deux extrémités.

7. Dispositif d'arrêt selon l'une quelconque des revendications précédentes, **caractérisé en ce que** la partie intermédiaire (16e) s'étend depuis la première partie (16c) dans le premier sens de rotation et/ou depuis la deuxième partie (16d) dans la direction opposée au premier sens de rotation.

8. Dispositif d'arrêt selon l'une quelconque des revendications précédentes, **caractérisé en ce qu'**un deuxième organe rotatif (3) est relié à l'élément de couplage (16) de telle sorte qu'il est poussé contre une première butée de rotation (16h) de l'élément de couplage (16) lorsqu'il tourne dans le premier sens de rotation, et est poussé contre une deuxième butée de rotation (16g) agissant dans le sens de rotation opposé lorsqu'il tourne dans le deuxième sens de rotation, ladite première butée de rotation (16h) étant formée sur la deuxième partie (16d) et ladite deuxième butée de rotation (16g) étant formée sur la première partie (16c) ou la partie intermédiaire (16e).

9. Dispositif d'arrêt selon l'une quelconque des revendications précédentes, **caractérisé en ce que** la première partie (16c) et/ou la deuxième partie (16d) comportent une structure anti-rotation (16a, 16b).

10. Dispositif de commande et/ou de dosage pour un dispositif d'injection permettant d'administrer un produit liquide, en particulier un médicament, comportant :
- un deuxième organe rotatif (3), en particulier un organe à emmancher, accessible en particulier par l'utilisateur du dispositif, et un ressort (11) extensible sous l'effet de la rotation du deuxième organe rotatif (3), ledit ressort (11) emmagasinant l'énergie nécessaire pour la distribution du produit,
**caractérisé par**
- un dispositif d'arrêt selon l'une quelconque des revendications précédentes, ledit dispositif d'arrêt étant disposé, en particulier cinématiquement entre le deuxième organe rotatif (3) et le ressort (11).

11. Dispositif de commande et/ou de dosage selon la revendication précédente, **caractérisé en ce qu'**une dose de produit à administrer peut être réglée par le dispositif de commande et/ou de dosage au moyen de la rotation du deuxième organe rotatif (3), comportant en outre un élément d'affichage de la dose (10) qui est couplé au deuxième organe rotatif (3) de telle sorte qu'une rotation du deuxième organe rotatif (3) induit une rotation de l'élément d'affichage de la dose (10).

12. Dispositif de commande et/ou de dosage selon l'une quelconque des deux revendications précédentes, **caractérisé par** un premier couplage (2b, 16b) qui est disposé, en particulier cinématiquement, entre l'élément de couplage (16) et le ressort de distribution (11), et par un organe d'actionnement (7) actionnable pour la distribution du produit, le premier couplage (2b, 16b) étant fermé lorsque l'organe d'actionnement (7) n'est pas actionné, et étant ouvert lorsque l'organe d'actionnement (7) est actionné ou étant ouvert sous l'effet de l'actionnement de l'organe d'actionnement (7).

13. Dispositif de commande et/ou de dosage selon la revendication précédente, **caractérisé en ce que** le premier couplage (2b, 16b) fermé relie un premier organe rotatif (2), en particulier un organe de couplage, disposé entre le ressort de distribution (11) et l'élément de couplage (16), de manière solidaire en rotation avec l'élément de couplage (16), ledit premier organe rotatif (2), en présence du premier couplage (2b, 16b) fermé, pouvant être entraîné en rotation par rapport à un organe de poussée (8), en particulier une tige de piston, agissant sur un piston d'un récipient à produit (14).

14. Dispositif de commande et/ou de dosage selon l'une quelconque des quatre revendications précédentes, **caractérisé par** un organe de poussée (8) allongé, dont l'extrémité distale peut agir en particulier sur un piston d'un récipient à produit (14), et dont le déplacement dans la direction distale induit un déplacement du piston à l'intérieur du récipient à produit (14), ledit organe de poussée (8) étant relié ou pouvant être relié à un premier organe rotatif (2), disposé entre l'élément de couplage (16) et le ressort de commande (11), de telle sorte qu'une rotation du premier organe rotatif (2) induit un mouvement de l'organe de poussée (8) dans la direction distale.

15. Dispositif de commande et/ou de dosage selon la revendication précédente, **caractérisé par** un organe d'actionnement (7), actionnable pour la distribution du produit, et un deuxième couplage (1a, 2a) qui est ouvert lorsque l'organe d'actionnement (7) n'est pas actionné et qui est fermé lorsque l'organe d'actionnement (7) est actionné ou est fermé sous l'effet de l'actionnement de l'organe d'actionnement (7), le deuxième couplage 1a, 2a) fermé reliant le premier organe rotatif (2) et l'organe de poussée (8) l'un à l'autre, de telle sorte qu'une rotation du premier organe rotatif (2) induit le mouvement de l'organe de poussée (8) dans la direction distale.
